# EUROPEAN PATENT SPECIFICATION

(11) **EP 1 495 052 B1**
(45) Date of publication and mention of the grant of the patent: **29.10.2008**
(21) Application number: 03746308.0
(22) Date of filing: 17.04.2003
(51) Int. Cl.: C07K 14/755, A61K 38/37

(54) **MODIFIED FACTOR VIII**
MODIFIZIERTER FAKTOR VIII
FACTEUR VIII MODIFIE

(30) Priority: 18.04.2002 EP 02008712; 24.03.2003 EP 03006554
(43) Date of publication of application: 12.01.2005
(73) Proprietor: Merck Patent GmbH, 64293 Darmstadt (DE)
(72) Inventor: JONES, Tim, Babraham, Cambridge CB2 4AJ (GB); BAKER, Matthew, Cambridge CB2 2RP (GB); CARR, Francis, J., Balmedie, Aberdeenshire AB23 8XU (GB)
(86) International application number: PCT/EP2003/004063
(87) International publication number: WO 2003/087161

(56) References cited:
- WO-A-00/34317
- WO-A-97/03195
- WO-A-98/52976
- WO-A-98/59244
- WO-A-99/46274
- WO-A-99/53038
- WO-A-02/090542
- WO-A-02/096454
- ALTUVIA Y ET AL: "RANKING POTENTIAL BINDING PEPTIDES TO MHC MOLECULES BY A COMPUTATIONAL THREADING APPROACH" JOURNAL OF MOLECULAR BIOLOGY, LONDON, GB, vol. 249, no. 1, 1995, pages 244-250, XP000925520 ISSN: 0022-2836
- MEISTER G E ET AL: "Two novel T cell epitope prediction algorithms based on MHC-binding motifs;comparison of predicted and published epitopes from Mycobacterium tuberculosis and HIV protein sequences" VACCINE, BUTTERWORTH SCIENTIFIC. GUILDFORD, GB, vol. 13, no. 6, 1 April 1995 (1995-04-01), pages 581-591, XP004057605 ISSN: 0264-410X
- BRUSIC VLADIMIR ET AL: "MHCPEP, a database of MHC-binding peptides: Update 1997" NUCLEIC ACIDS RESEARCH, OXFORD UNIVERSITY PRESS, SURREY, GB, vol. 26, no. 1, 1 January 1998 (1998-01-01), pages 368-371, XP002204283 ISSN: 0305-1048

## Description

### FIELD OF THE INVENTION

The invention in particular relates to the modification of human Factor VIII (FVIII) to result in FVIII proteins that are substantially non-immunogenic or less immunogenic than any non-modified counterpart when used *in vivo*.

### Background of the invention

There are many instances where the efficacy of a therapeutic protein is limited by an unwanted immune reaction to the therapeutic protein. Several mouse monoclonal antibodies have shown promise as therapies in a number of human disease settings but in certain cases have failed due to the induction of significant degrees of a human anti-murine antibody (HAMA) response [Schroff, R. W. et al (1985) Cancer Res. 45: 879-885; Shawler, D.L. et al (1985) J. Immunol. 135: 1530-1535]. For monoclonal antibodies, a number of techniques have been developed in attempt to reduce the HAMA response [WO-89/09622; EP 0239400; EP 0438310; WO 91/06667], These recombinant DNA approaches have generally reduced the mouse genetic information in the final antibody construct whilst increasing the human genetic information in the final construct. Notwithstanding, the resultant "humanized" antibodies have, in several cases, still elicited an immune response in patients [Issacs J.D. (1990) Sem, Immunol. 2: 449, 456; Rebello, P.R. et al (1999) Transplantation 68: 1417-1420].

Antibodies are not the only class of polypeptide molecule administered as a therapeutic agent against which an immune response may be mounted. Even proteins of human origin and with the same amino acid sequences as occur within humans can still induce an immune response in humans, Notable examples include the therapeutic use of granulocyte-macrophage colony stimulating factor [Wadhwa, M. et al (1999) Clin. Cancer Res. 5: 1353-1361] and interferon alpha 2 [Russo, D. et al (1996) Bri. J. Haem. 94: 300-305; Stein, R. et al (1988) New Engl. J. Med. 318: 1409-1413]. In such situations where these human proteins are immunogenic, there is a presumed breakage of immunological tolerance that would otherwise have been operating in these subjects to these proteins.

This situation is different where the human protein is being administered as a replacement therapy, for example in a genetic disease where there is a constitutional lack of the protein such as can be the case for diseases such as haemophilia A, Christmas disease, Gauchers disease and numerous other examples. In such cases, the therapeutic replacement protein may function immunologically as a foreign molecule from the outset, and where the individuals are able to mount an immune response to the therapeutic, the efficacy of the therapy is likely to be significantly compromised.

Irrespective of whether the protein therapeutic is seen by the host immune system as a foreign molecule, or if an existing tolerance to the molecule is overcome, the mechanism of immune reactivity to the protein is the same. Key to the induction of an immune response is the presence within the protein of peptides that can stimulate the activity of T-cells via presentation on MAC class II molecules, so-called "T-cell epitopes". Such T-cell epitopes are commonly defined as any amino acid residue sequence with the ability to bind to MHC class II molecules. Implicitly, a "T-cell epitope" means an epitope which when bound to MHC molecules can be recognised by a T-cell receptor (TCR), and which can, at least in principle, cause the activation of these T-cells by engaging a TCR to promote a T-cell response.

MHC Class II molecules are a group of highly polymorphic proteins which play a central role in helper T-cell selection and activation. The human leukocyte antigen group DR (HLA-DR) are the predominant isotype of this group of proteins however, isotypes HLA-DQ and HLA-DP perform similar functions. In the human population, individuals bear two to four DR alleles, two DQ and two DP alleles. There are approximately 70 different allotypes of the DR isotype, 30 different allotypes for DQ and 47 different allotypes for DP. The structure of a number of DR molecules has been solved and these appear as an open-ended peptide binding groove with a number of hydrophobic pockets which engage hydrophobic residues (pocket residues) of the peptide [Brown et al Nature (1993) 364: 33; Sterm et al (1994) Nature 368: 215]. The MHC DR molecules is made of an alpha and a beta chain which insert at their C-termini through the cell membrane. Each hetero-dimer possesses a ligand binding domain which binds to peptides varying between 9 and 20 amino acids in length, although the binding groove can accommodate a maximum of 11 amino acids. DQ molecules have recently been shown to have an homologous structure and the DP family proteins are expected to be very similar. Polymorphism identifying the different allotypes of the class II molecule contributes to a wide diversity of different binding surfaces for peptides within the peptide binding groove and at the population level ensures maximal flexibility with regard to the ability to recognise foreign proteins and mount an immune response to pathogenic organisms.

An immune response to a therapeutic protein proceeds via the MHC class II peptide presentation pathway. Here exogenous proteins are engulfed and processed for presentation in association with MHC class II molecules of the DR, DQ or DP type. MHC Class II molecules are expressed by professional antigen presenting cells (APCs), such as macrophages and dendritic cells amongst others. Engagement of an MAC class II peptide complex by a cognate T-cell receptor on the surface of the T-cell, together with the cross-binding of certain other co-receptors such as the CD4 molecule, can induce an activated state within the T-cell, Activation leads to the release of cytokines further activating other lymphocytes such as B cells to produce antibodies or activating T killer cells as a full cellular immune responses.

T-cell epitope identification is the first step to epitope elimination, however there are few clear cases in the art where epitope identification and epitope removal are integrated into a single scheme. Thus WO98/52976 and WO00/34317 teach computational threading approaches to identifying polypeptide sequences with the potential to bind a sub-set of human MHC class II DR allotypes. In these teachings, predicted T-cell epitopes are removed by judicious amino acid substitution within the protein of interest. However with this scheme and other computationally based procedures for epitope identification [e.g. Godkin, A.J. et al (1998) J. Immunol. 161: 850-858; Sturniolo, T. et al (1999) Nat. Biotechnol, 17: 555-561], peptides predicted to be able to bind MHC class II molecules may not function as T-cell epitopes in all situations, particularly *in vivo* due to the effects of the processing pathways or other phenomena. In addition, the computational approaches to T-cell epitope prediction have in general not been capable of predicting epitopes with DP or DQ restriction although in general there is overlap in recognition between these systems.

Besides computational techniques, there are *in vitro* methods for measuring the ability of synthetic peptides to bind MAC class II molecules. An exemplary method uses B-cell lines of defined MHC allotype as a source of MHC class II binding surface and may be applied to MHC class II ligand identification [Marshall, K.W. et al (1994) J. Immunol. 152: 4946-4956; O'Sullivan et al (1990) J. Immunol. 145: 1799-1808; Robadey, C. et al (1997) J. Immunol. 159: 3238-3246]. However, such techniques are not adapted for the screening ofmultiple potential epitopes against a wide diversity ofMHC allotypes, nor can they confirm the ability of a binding peptide to function as a T-cell epitope.

Recently techniques exploiting soluble complexes of recombinant MHC molecules in combination with synthetic peptides have come into use [Kern, F. et al (1998) Nature Medicine 4:975-978; Kwok, W.W, et al (2001) TRENDS in Immunol. 22:583-588]. These reagents and procedures are used to identify the presence of T-cell clones from peripheral blood samples from human or experimental animal subjects that are able to bind particular MHC-peptide complexes. These procedures also are not readily adapted for the screening of multiple potential epitopes to a wide diversity of MHC allotypes.

Biological assays of T-cell activation offer a practical option for providing a reading of the ability of a test peptide, or whole protein sequence, to evoke an immune response Examples of this kind of approach include the work of Petra et al using T-cell proliferation assays to the bacterial protein staphylokinase, followed by epitope mapping using synthetic peptides to stimulate T-cell lines (Petra. A.M. et al (2002) J. Immunol. 168: 155-161]. Similarly, T-cell proliferation assays using synthetic peptides of the tetanus toxin protein have resulted in definition of immunodominant regions of the toxin [Reece, J.C. et al (1993) J. Immunol. 151: 6175-6184]. WO99/53038 discloses an approach whereby T-cell epitopes in a test protein may be determined using isolated sub-sets of human immune cells, promoting their differentiation *in vitro* and culture of the cells in the presence of synthetic peptides of interest and measurement of any induced proliferation in the cultured T-cells. The same technique is also described by Stickler et al [Stickler, M.M. et al (2000) J. Immunotherapy 23: 654-660], where in both instances the method is applied to the detection of T-cell epitopes within bacterial subtilisin. Such a technique requires careful application of cell isolation techniques and cell culture with multiple cytokine supplements to obtain the desired immune cell sub-sets (dendritic cells, CD4+ and or CD8+ T-cells) and is not conducive to rapid through-put screening using multiple donor samples.

As depicted above and as consequence thereof it would be desirable to identify and to remove or at least to reduce T-cell epitopes from a given in principal therapeutically valuable but originally immunogenic peptide, polypeptide or protein. One of these therapeutically valuable molecules is FVIII. The present invention provides for modified forms of human FVIII with one or more T cell epitopes removed.

FVIII is a coagulation factor within the intrinsic pathway of blood coagulation. FVIII is a cofactor for factor IXa that, in the presence of calcium ions and phospholipid, converts factor X to the activated form Xa. The molecular genetics of FVIII are well studied not least as defects in the X-linked gene for FVIII give rise to haemophilia A. The FVIII gene encodes two alternatively spliced transcripts giving rise to the large glycoprotein FVIII isoform A and the smaller isoform B. In the native state multiple degradation or processed forms derived from the isoform A precursor can be identified and particular functional activities have been ascribed to particular fragments. The FVIII molecule is divided into 6 structural domains; a triplicated A domain (A1, A2, A3), a carbohydrate-rich and dispensable central domain (B-domain), and a duplicated C domain (C1, C2). FVIII proteolytic cleavage sites for thrombin and factor Xa occur after residues 372, 740 and 1689. Cleavage by Xa in conjunction with activated protein C occurs after residue 336.

FVIII protein may be functionally defined as a factor capable of correcting the coagulation defect in plasma derived from patients affected by haemophilia A. For the treatment of haemophilia A, FVIII has been produced in purified form from human or porcine plasma and more recently by recombinant DNA technologies. The human FVIII gene was isolated and expressed in mammalian cells by at least 2 independent laboratory groups in 1984 [Toole, J. J., et al. (1984), Nature 312 342-347; Gitschier, J., et al. (1984), Nature 312: 326-330; Wood, W. I., et al.(1984), Nature 312 330-337; Vehar, G. A., et al. (1984), Nature 312: 337-342; WO87/04187; WO88/08035; WO88/03558; US,.4,757,006]. The amino acid sequence was deduced from cDNA and methods for the recombinant production of therapeutic quantities of FVIII have been developed, for example US,4,965,199 discloses a method for producing FVIII in mammalian host cells and purification of human FVIII. Human FVIII expression in CHO (Chinese hamster ovary) cells and BHKC (baby hamster kidney cells) has been reported and more recently FVIII has been modified to delete part or all of the B domain [U.S. Pat. No. 4,868,112] and such a molecule has shown efficacy in clinical trials [Lusher, J.M. et al (2003) Haemophilia 9: 38-49]. This molecules is in fact licensed for use - Refacto is made under a collaboration between the Genetics Institute and Pharmacia Upjohn in CHO cells.

Despite the availability of therapeutic quantities of FVIII, there is a continued need for FVIII analogues with enhanced properties. Desired enhancements include alternative schemes and modalities for the expression and purification of the protein, but also and especially, improvements in the biological properties of the protein. There is a particular need for enhancement of the *in vivo* characteristics when administered as a therapeutic. In this regard, it is highly desired to provide FVIII with reduced or absent potential to induce an immune response in the human subject. Such proteins would expect to display an increased circulation time within the human subject and would be of particular benefit in the chronic and recurring disease setting such as is the case haemophilia A in particular in cases where haemophiliacs may be sensitive to exogenous recombinant factor VIII and would otherwise develop anti-actor VIII antibodies which would limit the effectiveness of their treatment

Others have provided FVIII molecules and in particular recombinant modified FVIII [US 4,757,006; US 5,633,150; US 5,668,108], but none of these teachings recognise the importance of T cell epitopes to the immunogenic properties of the protein nor have been conceived to directly influence these properties in a specific and controlled way according to the scheme of the present invention.

Non-exhaustive examples in the art for the modifications of FVIII include US 5,948,407 wherein are disclosed schemes for producing formulations to enable mucosal (e.g. oral) administration of the protein for the purpose of evoking a suppressor T cell repertoire to the therapeutic protein antigen.

Others have attempted modifications to FVIII for the purpose of improving *in vivo* half life in the haemophiliac subject, for example US 6,037,452 describes poly(alkylene oxide) factor VIII conjugates.

A particular complication in the therapy of haemophilia A is the induction in certain patients of inhibitory antibodies to the administered FVIII preparation. Several strategies have been advanced to overcome the immunological response to FVIII in haemophiliacs. Tolerance induction therapy is one such approach but currently requires very large (and costly) doses of FVIII preparation to be administered very early in the life of a haemophiliac. The approach is only successful in achieving tolerance in a proportion cases [Colowick, A.B. et al (2000) Blood 96: 1698-1702]. Clinical trials of two different recombinant products in previously untreated subjects reported inhibitor development in around 20% of cases [Lusher, J.M. et al (1993) N.Engl. J. Med 328:453-459; Bray, G.L. et al (1994) Blood 83: 2428-2435]. FVIII inhibitors are immunoglobulin antibodies that neutralise FVIII activity in plasma. The inhibitors arise as alloantibodies in approximately 25% of patients with severe haemophilia A and 5-15% of patients with mild to moderate disease who are treated with FVIII concentrates or recombinant FVIII.

For patients with inhibitors treatment with factor IX complex or "prothrombin complex" is also used. In general these are mixed preparations of multiple different factors including for example Factors II, VII, IX and X. EP0044343-B1 provides such a prothrombin, complex featuring an activated component (FVIIa). Similarly, US 6,358,534 describes an immunotolerant prothrombin complex comprising a low proportion of FVIII protein and relatively higher proportions of other clotting protein factors and carrier proteins.

US 5,543,145 provides compositions for the suppression of FVIII inhibitor production comprising polyclonal or monoclonal antibodies for example in the form of F(ab')₂ mixed with FVIII. US 4,769,336 provides FVIII fragments which bind and thereby block antibody inhibitors of FVIII.

Other strategies include use of porcine factor VIII in place of human FVIII however such treatment is a temporary measure as porcine FVIII is itself immunogenic.
Other options available include treatment with immune suppressive drugs and or removal of inhibitors from the circulation by extracorporeal treatments and understandably such approaches represent extreme and hazardous solutions to the problem at hand.
Genetic engineering techniques have provided considerable scope for the modification and indeed production of FVIII molecules for the therapeutic use. Pharmaceutical compositions and treatment of haemophilia A is provided by the teachings of US 4,965,199; US 5,618,788 and US 5,668,108 and foreign equivalents and should be considered exemplary of a large body of works on this subject available in the art.

Further exemplary types of modification to FVIII conducted using recombinant approaches are provided in US 5,859,204; US 6,376,463; US 6,485,563 and US 6,180,371 which collectively provide FVIII proteins and their encoding genes in which site-specific substitutions have resulted in reduction of reactivity to an inhibitory antibody. In these particular teachings the substitutions are confined to amino acids 484-509 of the FVIII A domain. Such modifications whilst changing the surface topology of the FVIII molecule such that particular conformational epitopes are no longer recognisable by particular cross-reacting antibodies in the patient serum, do not address the underlying T-cell epitopes required to drive B-cell production of antibodies.

Others have used recombinant DNA means to provide FVIII molecules modified using by site-specific amino acid substitutions [WO87/07144] or swapping of domains between related molecules. An example of the latter approach is provided by WO90/05530 wherein is provided a FVIII molecule containing the B-domain of human factor V in place of the FVIII B-domain.
Others still have provided chimaeric FVIII preparations comprising recombinant FVIII molecules engineered to contain human and porcine (or other species) derived sequence domains. Such hybrid molecules are described in US 5,744,446; US 5,663,060; US 5,583,209; US 5,364,771; US 5,888,974 and elsewhere.
US 5,171,844 provides genetically engineered FVIII molecules which may have enhanced activity and or decreased immunogenicity. The latter property related to absence of sections of the molecule by deletion. Earlier work described in US 4,868,112 and foreign equivalents provides functional deletion mutants of FVIII lacking the B-domain.
Other genetic engineering modifications to the FVIII protein have been directed towards improvements in the production process for the protein and particular examples are provided by US 6,271,025. This example is provided as being exemplary of a large body of further similar works available and known in the art.

### SUMMARY AND DESCRIPTION OF THE INVENTION

The present invention provides for modified forms of human factor VIII, herein called "FVIII", in which the immune characteristic is modified by means of reduced or removed numbers of potential T-cell epitopes.

The invention discloses sequences identified within the FVIII primary sequence that are potential T-cell epitopes by virtue of MHC class II binding potential. This disclosure specifically pertains equally to the complete human FVIII protein being isoform A of 2332 amino acid residues and a smaller version in which the B-domain is absent from the protein.

The invention discloses also specific positions within the primary sequence of the molecule which according to the invention are to be altered by specific amino acid substitution, addition or deletion whilst retaining to a maximum degree the biological activity of the protein. In cases in which the loss of immunogenicity can be achieved only by a simultaneous loss of biological activity it is possible to restore the activity by further alterations within the amino acid sequence of the protein.

The invention furthermore discloses methods to produce such modified molecules, and above all methods to identify the T-cell epitopes which require alteration in order to reduce or remove immunogenic sites.

The present invention provides for modified forms of FVIII proteins that are expected to display enhanced properties *in vivo.* The present invention discloses the major regions of the FVIII primary sequence that are immunogenic in man and provides modification to the sequences to eliminate or reduce the immunogenic effectiveness of these sites.
In one embodiment, synthetic peptides comprising the immunogenic regions can be provided in pharmaceutical composition for the purpose of promoting a tolerogenic response to the whole molecule.

In a further embodiment, the modified FVIII molecules of the present invention can be used in pharmaceutical compositions.

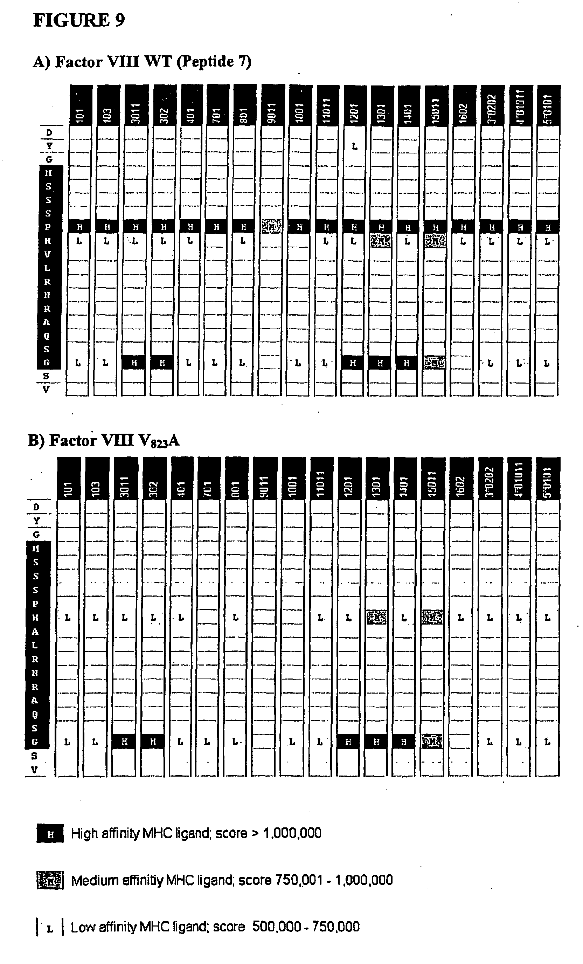

In summary the invention relates to the following issues:
- a modified molecule having the biological activity of FVIII and being substantially non-immunogenic or less immunogenic than any non-modified molecule having the same biological activity when used *in vivo*;
- an accordingly specified molecule, wherein said loss of immunogenicity is achieved by removing one T-cell epitope derived from the originally non-modified molecule;
- an accordingly specified molecule, wherein said loss of immunogenicity is achieved by reduction in numbers of MHC allotypes able to bind a peptide derived from said molecule;
- an accordingly specified molecule, wherein one T-cell epitope is removed;
- an accordingly specified molecule, wherein said originally present T-cell epitopes are MHC class II ligands or peptide sequences which show the ability to stimulate or bind T-cells via presentation on class II;
   a) an accordingly specified molecule, wherein said peptide sequence is MSSSPHVLRNRAQSG,
- an accordingly specified molecule, wherein one amino acid residue the originally present T-cell epitope is altered;
- an accordingly specified molecule, wherein the alteration of the amino acid residues is substitution of originally present amino acid(s) residue(s) by other amino acid residue(s) at specific position(s);
- an accordingly specified molecule, wherein, if necessary, additionally further alteration usually by substitution, addition or deletion of specific amino acid(s) is conducted to restore biological activity of said molecule;
- an accordingly specified FVIII molecule, wherein the amino acid substitution is conducted at position V7 of the above peptide corresponding to position V823 in the wild-typ sequence according to B domain deleted sequence (Figur 9);
- a pharmaceutical composition comprising any of the peptides or modified peptides of above having the activity of binding to MHC class II
- a DNA sequence or molecule which codes for any of said specified modified molecules as defined above and below;
- a pharmaceutical composition comprising a modified molecule having the biological activity of FVIII
- a pharmaceutical composition as defined above and / or in the claims, optionally together with a pharmaceutically acceptable carrier, diluent or excipient;

The term "T-cell epitope" means according to the understanding of this invention an amino acid sequence which is able to bind MHC class II, able to stimulate T-cells and / or also to bind (without necessarily measurably activating) T-cells in complex with MHC class II.

The term "peptide" as used herein and in the appended claims, is a compound that includes two or more amino acids. The amino acids are linked together by a peptide bond (defined herein below). There are 20 different naturally occurring amino acids involved in the biological production of peptides, and any number of them may be linked in any order to form a peptide chain or ring. The naturally occurring amino acids employed in the biological production of peptides all have the L-configuration. Synthetic peptides can be prepared employing conventional synthetic methods, utilizing L-amino acids, D-amino acids, or various combinations of amino acids of the two different configurations. Some peptides contain only a few amino acid units. Short peptides, e.g., having less than ten amino acid units, are sometimes referred to as "oligopeptides". Other peptides contain a large number of amino acid residues, e.g. up to 100 or more, and are referred to as "polypeptides". By convention a "polypeptide" may be considered as any peptide chain containing three or more amino acids, whereas a "oligopeptide" is usually considered as a particular type of "short" polypeptide. Thus, as used herein" it is understood that any reference to a "polypeptide" also includes an oligopeptide. Further, any reference to a "peptide" includes polypeptides, oligopeptides, and proteins. Each different arrangement of amino acids forms different polypeptides or proteins. The number of polypeptides-and hence the number of different proteins-that can be formed is practically unlimited. "Alpha carbon (Cα)" is the carbon atom of the carbon-hydrogen (CH) component that is in the peptide chain. A "side chain" is a pendant group to Cα that can comprise a simple or complex group or moiety, having physical dimensions that can vary significantly compared to the dimensions of the peptide.

The invention may be applied to any FVIII species of molecule with substantially the same primary amino acid sequences as those disclosed herein and would include therefore FVIII molecules derived by genetic engineering means or other processes and may contain more or less than 2332 amino acid residues.

A particularly preferred FVIII species of molecule is one in which the B-domain of the molecule is lacking and yet which also comprises one or more amino acid substitutions in any of the remaining domains to result in the elimination from the sequence of one or more T-cell epitopes. A therapeutic B-domain deleted FVIII molecule of 1438 amino acid residues has been the subject of successful clinical trials as described by Lusher et al and references therein [Lusher, J.M. et al (2003) Haemophilia 9: 38-49]. The amino acid sequence of such a protein is depicted as FIGURE 9 herein.

FVIII proteins such as identified from other mammalian sources have in common many of the peptide sequences of the present disclosure and have in common many peptide sequences with substantially the same sequence as those of the disclosed listing. Such protein sequences equally therefore fall under the scope of the present invention.

The invention is conceived to overcome the practical reality that soluble proteins introduced into autologous organisms can trigger an immune response resulting in development of host antibodies that bind to the soluble protein. For many patients with haemophilia A, antibody responses to the existing therapeutic preparations of FVIII is a significant problem in the management of their disease. In any individual a small number of T-cell epitopes can drive T-helper cell signalling to result in sustained antibodiy responses to the very many B-cell recognised surface exposed determinants of the native FVIII protein, Analysis of the genes encoding inhibitor antibodies from haemophilia A patients has shown that many of such antibodies to have undergone affinity maturation [Jacquemin, M.G. et al (1998) Blood 92: 496-506; van den Brink E.N et al (2000) Blood 95: 558-563]. The hypermutation phenomenon underlying affinity maturation occurs only in B-cells in response to T-cell help. It is also long recognised that a high proportion of FVIII inhibitor antibodies are of the IgG4 subclass [Andersen, B.R. & Terry, W.D. (1968) Nature 217: 174-175] and the necessary class switching reactions are similarly T helper cell dependent events. Further evidence that the antibody response to FVIII is T-cell driven comes also from the observation that in HIV positive patients with FVIII inhibitors, the FVIII antibody response is lost as T-cell numbers decline [Bray, G.L. et al (1993) Am. J. Hematol. 42: 375-379]. Accordingly, the present invention seeks to address this by providing FVIII proteins with altered propensity to elicit an immune response on administration to the human host. There is an understanding that where the multiplicity of B-cell epitopes remain intact on the surface of the modified FVIII, in the absence of continued T-cell help the inhibitor titres will ultimately decrease and for new patients receiving such a therapy fail to become established in the first instance. According to the methods described herein, the inventors have discovered the regions of the FVIII molecule comprising the critical T-cell epitopes driving the immune responses to this protein.

The general method of the present invention leading to the modified FVIII comprises the following steps;
(a) determining the amino acid sequence of the polypeptide or part thereof;
(b) identifying one or more potential T-cell epitopes within the amino acid sequence of the protein by any method including determination of the binding of the peptides to MHC molecules using *in vitro* or *in silico* techniques or biological assays;
(c) designing new sequence variants with one or more amino acids within the identified potential T-cell epitopes modified in such a way to substantially reduce or eliminate the activity of the T-cell epitope as determined by the binding of the peptides to MHC molecules using *in vitro* or *in silico* techniques or biological assays. Such sequence variants are created in such a way to avoid creation of new potential T-cell epitopes by the sequence variations unless such new potential T-cell epitopes are, in turn, modified in such a way to substantially reduce or eliminate the activity of the T-cell epitope; and
(d) constructing such sequence variants by recombinant DNA techniques and testing said variants in order to identify one or more variants with desirable properties according to well known recombinant techniques.
The identification of potential T-cell epitopes according to step (b) can be carried out according to methods described previously in the art. Suitable methods are disclosed in WO 98/59244; WO 98/52976; WO 00/34317; WO 02/069232 and may be used to identify binding propensity of FVIII-derived peptide to an MHC class II molecule. In practice, the compositions embodied in the present invention have been derived with the concerted application of biological ex vivo human T-cell proliferation assays and a software tool exploiting the scheme outlined in WO 02/069232 and which is an embodiment of the present invention.
The software simulates the process of antigen presentation at the level of the peptide MHC class II binding interaction to provide a binding score for any given peptide sequence. Such a score is determined for many of the predominant MAC class II allotypes extant in the population. As this scheme is able to test any peptide sequence, the consequences of amino acid substitutions additions or deletions with respect to the ability of a peptide to interact with a MHC class II binding groove can be predicted. Consequently new sequence compositions can be designed which contain reduced numbers of peptides able to interact with the MHC class II and thereby function as immmunogenic T-cell epitopes. Where the biological assay using any one given donor sample can assess binding to a maximum of 4 DR allotypes, the *in silico* process can test the same peptide sequence using >40 allotypes simultaneously. In practice this approach is able to direct the design of new sequence variants which are compromised in the their ability to interact with multiple MHC allotypes.

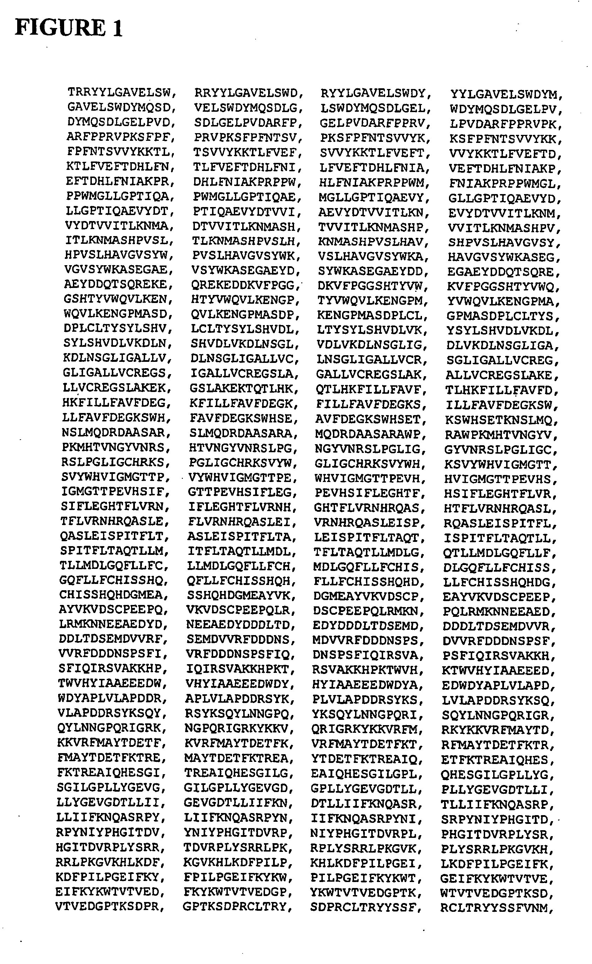

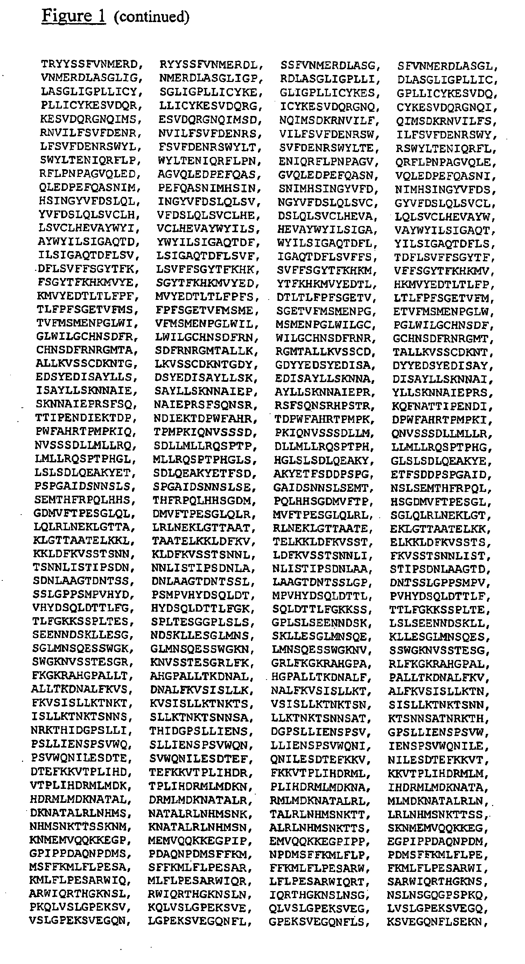

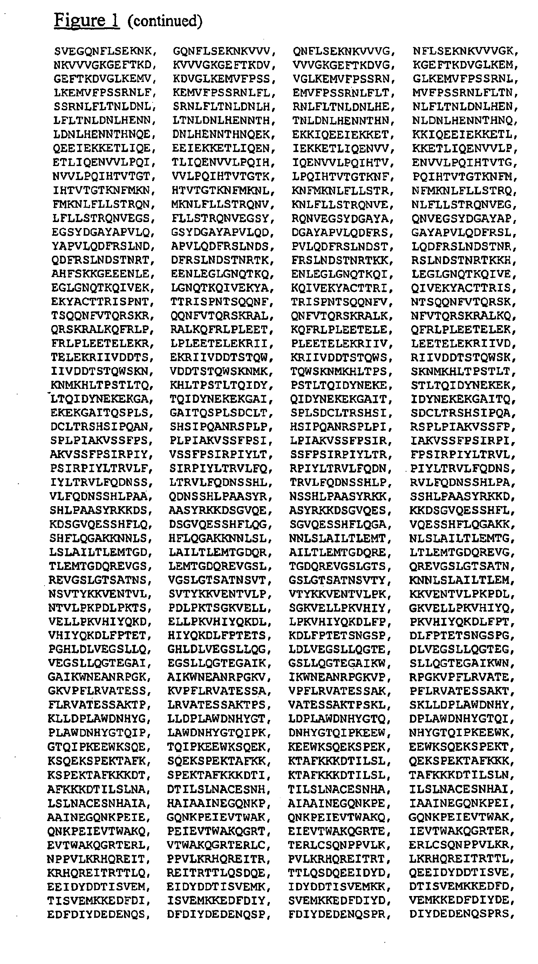

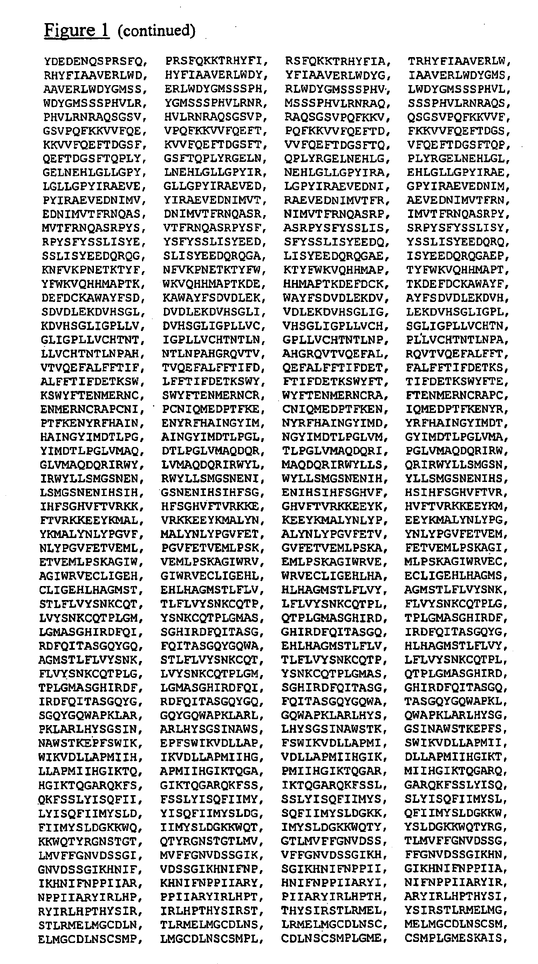

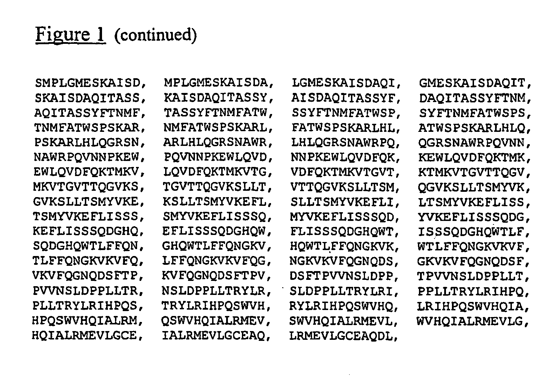

By way of an example of this *in silico* approach, the results of an analysis conducted on the entire human FVIII sequence is provided as FIGURE 1. Therein are listed 13mer peptide sequences derived from FVIII detected to have the capability to bind one or more MHC class II allotypes with a significant binding score. Taken in its entirety, this dataset of 13mer peptides is considered to provide with a high degree of certainty, the universe of permissible MHC class ligands for the human FVIII protein. For reasons such as the requirement for proteolytic processing of the complete FVIII polypeptide and other physiologic steps leading to the presentation of FVIII peptides *in vivo*, it would be clear that a relatively minor sub-set of the entire repertoire of peptides will have ultimate biological relevance. In order to further identify such biologically relevant peptides, the inventors have developed an approach exploiting *ex vivo* human T-cell proliferation assays.

This approach has proven to be a particularly effective method and is disclosed herein as an embodiment of the invention. The method can be applied to test part of the sequence, for example a FVIII protein lacking all or part of the B-domain; the method can be applied to selected regions of the sequence, for example a sub-set of FVIII peptides such as all or some of those listed in FIGURE 1; or the method may be applied to test whole FVIII sequence. In the present studies, the method has involved the testing of overlapping FVIII-derived peptide sequences in a scheme so as to scan and test a FVIII sequence lacking the B-domain. The synthetic peptides are tested for their ability to evoke a proliferative response in human T-cell cultured *in vitro*. Where this type of approach is conducted using naïve human T-cells taken from healthy donors, the inventors have established that in the operation of such an assay, a stimulation index equal to or greater than 2.0 is a useful measure of induced proliferation. The stimulation index is conventionally derived by division of the proliferation score (*e.g*. counts per minute of radioactivity if using ³H-thymidine incorporation) measured to the test peptide by the score measured in cells not contacted with a test peptide.

In a further embodiment of this approach, the inventors have provided a method whereby the FVIII sequence is scanned for the presence and location of T-cell epitopes using ex vivo biological T-cell assays where the T-cells are derived from haemophiliac patients. For a proportion of such patients the FVIII protein constitutes a foreign protein and a potent antigen *in vivo*. The inventors have established that it is possible to derive polyclonal and in principle mononclonal T-cell lines *in vitro* from the PBMC of such individuals and these lines may be used as effective reagents in the napping of T-cell epitopes within the FVIII protein.

This is achieved by subjecting haemophiliac PBMC T-cells to several rounds of antigen (FVIII) stimulation *in vitro* followed immediately by expansion in the presence of IL-2. For establishing polyclonal T cell lines 2-3 rounds of antigen stimulation were generally sufficient to generate a large number of antigen specific cells, and such cells may be stored cryogenically for subsequent use as required. The cells were used to screen large numbers of synthetic peptides,

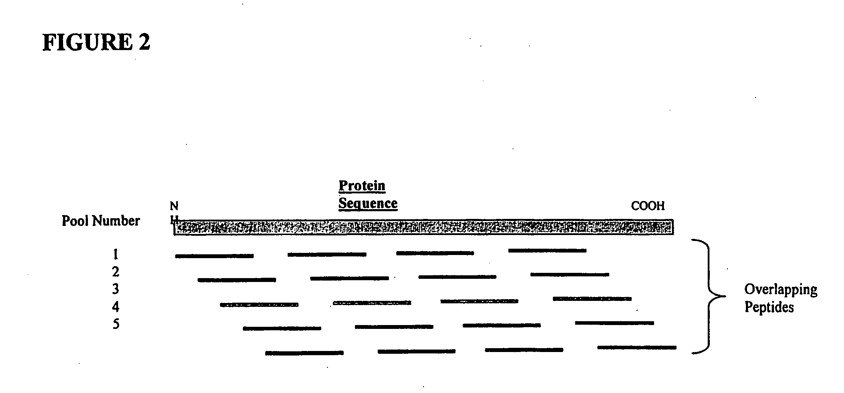

In the present case in the synthetic peptides were analysed using a system of pools each containing 8 different peptide sequences. The peptide pool scheme is illustrated in FIGURE 2. After the initial round of antigen (FVIII) stimulation comprising co-incubation of FVIII and PBMC for 7 days subsequent re-challenges with the antigen was performed in the presence of autologous irradiated PBMC as antigen presenting cells. These rounds of antigen selection are performed for 3-4 days and are interspersed by expansion phases comprising stimulation with IL-2 which may be added every 3 days for a total period of around 9 days. The final re-challenge is performed using T-cells that have been "rested", that is T cells which have not been IL-2 stimulated for around 4 days. These cells are stimulated with antigen (e.g. synthetic peptide or whole protein) using most preferably autologous antigen presenting cells as previously for around 4 days and the subsequent proliferative response (if any) is measured thereafter. The peptide pools are organised such that each pool contains overlapping peptides to the subsequent pool. In this way any individual T-cell epitope is represented in 2 separate pools and induced proliferation will be detected from treatment using both of those pools. Where a proliferative response is detected to any given peptide pool, the peptide pool is decoded repeating the assay using the individual pool members in isolation.

Under the scheme of the present it has been particularly desired to exploit PBMC samples from the class of so called "inhibitor patients" as it could be expected that the epitope map of the FVIII protein defined by the T-cell repertoire from these individuals will be representative of the most prevalent peptide epitopes that are capable of presentation in the *in vivo* context. In this sense, PBMC from patients in whom there is a previously demonstrated immune response constitute the products of an *in vivo* primping step and thee could be an expectation of a practical benefit in there being the capacity for a much larger magnitude of proliferative response to any given stimulating peptide. This reduces the technical challenge of conducting a proliferation measurement.

The present studies have uncovered some 38 peptide sequences able to evoke a significant proliferative response (i.e. SI>2.0). These peptides are listed in TABLE 1 and are an embodiment of the invention. Within this set of peptides, a further sub-set of peptides have been identified which evoke a significant proliferative response in 2 or more individual donor samples and for some of theses responses the magnitude of response has indeed been significantly higher than SI=2.0. These peptides are listed in TABLE 2 and are a further embodiment of the invention.

**TABLE 1:**

| **Peptides Sequence** | **Residue #*** |
|---|---|
| ATRRYYLGAVELSWD | 1 |
| SWDYMQSDLGELPVD | 13 |
| PVDARFPPRVPKSFP | 25 |
| SGVYKKTLFVEFTDH | 43 |
| VEFTDHLFNIAKPRP | 52 |
| PWMGLLGPTIQAEVY | 67 |
| LKNMASHPVSLHAVG | 88 |
| VSYWKASEGAEYDDQ | 103 |
| PGGSHTYVWQVLKEN | 130 |
| ASDPLCLTYSYLSHV | 148 |
| ILLFAVFDEGKSWHS | 196 |
| SLPGLIGCHRKSVYW | 241 |
| VYWHVIGMGTTPEVH | 253 |
| QASLEISPITFLTAQ | 283 |
| YIAAEEEDWDYAPLV | 385 |
| SYKSQYLNNGPQRIG | 406 |
| GPQRIGRKYKKVRFM | 415 |
| PYNIYPHGITDVRPL | 472 |
| YKWTVTVEDGPTKSD | 511 |
| KESVDQRGNQIMSDK | 556 |
| QIMSDKRNVILFSVF | 565 |
| PAGSVQLEDPEPQASN | 598 |
| ASNIMHSINGYVFDS | 610 |
| LQLSVCLHEVAYWYI | 625 |
| VAYWYILSIGAQTDF | 634 |
| GAQTDFLSVFFSGYT | 643 |
| GCHNSDFRNRGMTAL | 691 |
| TGDYYEDSYEDISAY | 712 |
| LLSKNNAIEPRSFSQ | 728 |
| MSSSPHVLRNRAQSG | 817 |
| NEHLGLLGPYIRAEV | 859 |
| AWAYFSDVDLEKDVH | 940 |
| CNIQMEDPTPKENYR | 1009 |
| IGEHLHAGMSTLFLV | 1108 |
| STLFLVYSNKCQTPL | 1117 |
| ISQFIIMYSLDGKKW | 1204 |
| IARYIRLHPTHYSIR | 1251 |
| RLHPTHYSIRSTLRM | 1256 |

| | |
|---|---|
| * Sequence numbering according to B domain deleted sequence (FIGURE 9) | |

**TABLE 2:**

| *FVIII peptide sequences able to stimulate ex vivo human T-cells from 2 or more donor samples* | | | |
|---|---|---|---|
| **Peptides No.** | **Residue #** | **Peptide Sequence** | **Domain** |
| P1 | 196 | ILLFAVFDEGKSWTSH | A1 |
| P2 | 406 | SYKSQYLNNGPQRIG | A2 |
| P3 | 415 | GPQRIGRKYKKVRFM | A2 |
| P4 | 511 | YKWTVTVRDGPTKSD | A2 |
| P5 | 610 | ASNIMHSINGYVFDS | A2 |
| P6 | 634 | VAYWY2LSTGAQTDF | A2 |
| P7 | 817 | MSSSPHVLRNRAQSG | A3 |
| P8 | 1009 | CNIQMEDPTFKENYR | A3 |
| P9 | 1117 | STLFLVYSNKCQTPL | A3 |
| P10 | 1204 | ISQFIIMYSLDGKKW | C1 |
| P11 | 1251 | IARYIRLHPTHYSIRSTLRM | C1 |

| | | | |
|---|---|---|---|
| * Sequence numbering according to B domain deleted sequence (FIGURE 9) | | | |

The disclosed peptide sequences herein represent the critical information required for the construction of modified FVIII molecules in which one or more of these epitopes is compromised. Under the scheme of the present, the epitopes are compromised by mutation to result in sequences no longer able to function as T-cell epitopes. It is possible to use recombinant DNA methods to achieve directed mutagenesis of the target sequences and many such techniques are available and well know in the art.

Where it is the objective of this invention to modify the amino acid sequences of at least one or more of the above listed peptides from TABLE 1, it is most preferred to modify the sequence of one or more of the peptides P1- P11 identified in TABLE 2. There are herein disclosed suitable modifications which achieve the dual objectives of reducing or eliminating the capabilities of the subject peptide sequence to function as a T-cell epitope, either at the level of being a ligand for one or more MHC class II allotypes, or more especially being able to induce T-cell, proliferation and especially where the T-cells are human T-cells cultured *in vitro*.

One example of such a set of preferred modifications is provided by the disruption of the T-cell epitope encompassed by peptide P10 with the sequence ISQFIIMYSLDGKKW. This epitope maps to the C1 domain of FVIII and is exemplary of an epitope able to evoke proliferation of *ex vivo* T-cells taken from both haemophiliac blood and the cells of a healthy non haemophiliac individual.

Guided by the results of *in silico* analysis of this peptide sequence with respect to its MHC class II ligand activity, site directed mutagenesis procedures have been applied to produce substitutions at residues F1207,11208, I1209 and M1210. Residue numbering is according to the mature B-domain deleted FVIII sequence (FIGURE 9). Mutagenesis at F1207 resulted in loss of FVIII expression and no detectable activity in the assays employed. In contrast active FVIII molecules were produced comprising substitutions I1208A or I1208T or I1208N and or I1209C and or M1210K or M1210N. Accordingly, active FVIII proteins with one or more of the above listed substitutions are preferred compositions under the scheme of the present. Particularly preferred substitutions are I1208A, I1209C, M1210K or M1210N and such substituted FVIII proteins are a further embodiment of the invention.

Similarly preferred embodiments comprise FVIII molecules containing substitutions within the T-cell epitope encompassed by peptide P8 with the sequence CNIQMEDPTFKENYR. This epitope maps to the A3 domain of the FVIII protein. Site directed mutagenesis procedures have been applied to this sequence and the substitutions M1013K, I1011A or C or D or E or G or H or K or P or Q or R or S or T have been established to provide a molecule with retained functional activity with respect to the coatest assay and immunological parameters *in silico* and immunological assays *in vitro.* FVIII molecules encompassing the substitutions M1013K, I1011A or C or D or E or G or H or K or P or Q or R or S or T are accordingly further embodiments of the invention.

Similarly preferred embodiments comprise FVIII molecules containing substitutions within the T-cell epitope encompassed by peptide P7 with the sequence MSSSPHVLRNRAQSG. This epitope also maps to the A3 domain of the FVIII protein. Substitution at position V823 is considered an especially desired modification and is an embodiment of the invention. Site directed mutagenesis procedures have been applied to this sequence and the substitutions V823A, or D or E or G or H or N or P or S or T have been established to provide a molecule with retained functional activity with respect to the coatest assay and immunological parameters *in silico* and immunological assays *in vitro*. FVIII molecules encompassing the substitutions V823A, or D or E or G or H or N or P or S or T are accordingly further embodiments of the invention.

A further example of a T-cell epitope peptide sequence shown to be active in haemophiliac blood samples is provided by the peptide P11 with the sequence IARYIRLHPTHYSTRSTLRM. This epitope maps to the C1 domain of the FVIII protein and has been identified previously as a significant driver of FVIII inhibitor production [Jacquemin, M. et al (2003) Blood 101:1351-1358]. Substitutions at positions Y1254, 11255, L1257, Y1262, I1264, L1268 are considered especially desired modifications and are an embodiment of the invention.

Further similarly preferred embodiments comprise FVIII molecules containing substitutions at positions L1119, F1120, L1121, V1122 and Y1123 of the epitope encompassed by peptide P9 (STLFLVYSNKCQTPL) also substitutions at positions Y636, Y638, I637, L638 and 1639 of the epitope encompassed by peptide P6 (VAYWYILSIGAQTDF); substitutions at positions I613 and I617 of the epitope encompassed by peptide P5 (ASNIMHSINGYVFDS); substitutions at positions V515 and V517 of the epitope encompassed by peptide P4 (YKWTVTVRDGPTKSD); substitutions at position I419 of the epitope encompassed by peptide P3 (GPQRIGRKYKKVRFM); substitutions at positions Y407, Y411 and L412 of the epitope encompassed by peptide P2 (SYKSQYLNNGPQRIG) and substitutions at positions L197, L198, F199, V201 and F202 of the epitope encompassed by peptide P1 (ILLFAVFDEGKSWSH).

From the foregoing it can be seen that according to this invention a number of variant FVIII proteins can be produced and tested for the desired immune and functional characteristic. Exemplary methods for the making and testing of such FVIII variant proteins are described within the EXAMPLES. All such functional proteins are embodiments of the present invention. Moreover the modifications conducted have been demonstrated to result in peptide sequences not able to bind MHC class II molecules with the same avidity as the parental or '"wild-type" (wt) peptide sequence using the predictive *in silico* MHC class II binding tool of WO02/069232. Furthermore, physical testing of the exemplary variant peptide epitopes using *ex vivo* human T-cell biological proliferation assays confirm the desired loss of ability for these peptides to support T-cell proliferation and thereby function as a T-cell epitope. In the present case this includes peptide sequences identified using biological assays and thereby confirmed to be capable of supporting T-cell activation. Significantly a number of such peptides are able to promote *in vitro* proliferation of T-cells derived from haemophiliac blood and are thereby are representative of processed epitopes and can not be considered cryptic or immunologically irrelevant determinants.

The variant proteins will most preferably be produced by recombinant DNA techniques although other procedures including chemical synthesis of FVIII fragments may be contemplated. It is a facile procedure to use the protein sequence information provided herein to deduce polynucleotides (DNA) encoding any of the preferred variant FVIII molecules or fragments. This is most readily achieved using computer software tools such as the DNAstar software suite [DNAstar Inc, Madison "WI, USA] or Similar, Any such DNA sequence encoding the polypeptide of the present or significant homologues thereof should be considered as embodiments of this invention.

The invention relates to FVIII analogues in which substitutions of at least one amino acid residue have been made at positions resulting in a substantial reduction in activity of or elimination of one or more potential T-cell epitopes from the protein. One or more amino acid substitutions at particular points within any of the potential MHC class II ligands identified in FIGURE 1 or more preferably peptide epitope sequences of TABLES 1 and 2 may result in a FVIII molecule with a reduced immunogenic potential when administered as a therapeutic to the human host.

It is most preferred to provide an FVIII molecule in which amino acid modification (e.g. a substitution) is conducted within the most immunogenic regions of the parent molecule. For the elimination of T-cell epitopes, amino acid substitutions are preferably made at appropriate points within the peptide sequence predicted to achieve substantial reduction or elimination of the activity of the T-cell epitope. In practice an appropriate point will preferably equate to an amino acid residue binding within one of the pockets provided within the MHC class II binding groove. It is most preferred to alter binding within the first pocket of the cleft at the so-called P1 or P1 anchor position of the peptide. The quality of binding interaction between the P1 anchor residue of the peptide and the first pocket of the MHC class II binding groove is recognised as being a major determinant of overall binding affinity for the whole peptide. An appropriate substitution at this position of the peptide will be for a residue less readily accommodated within the pocket, for example, substitution to a more hydrophilic residue. Amino acid residues in the peptide at positions equating to binding within other pocket regions within the MHC binding cleft are also considered and fall under the scope of the present invention.

As will be clear to the person skilled in the art, multiple alternative sets of substitutions could be arrived at which achieve the objective of removing un-desired epitopes. The resulting sequences would however be recognised to be closely homologous with the specific compositions disclosed herein and therefore fall under the scope of the present invention.

It is understood that single amino acid substitutions within a given potential T-cell epitope are the most preferred route by which the epitope may be eliminated. Combinations of substitution within a single epitope may be contemplated and for example can be particularly appropriate where individually defined epitopes are in overlap with each other. Moreover, amino acid substitutions either singly within a given epitope or in combination within a single epitope may be made at positions not equating to the "pocket residues" with respect to the MAC class II binding groove, but at any point within the peptide sequence. Substitutions may be made with reference to an homologues structure or structural method produced using *in silico* techniques known in the art and may be based on known structural features of the molecule according to this invention. All such substitutions fall within the scope of the present invention.

Amino acid substitutions other than within the peptides identified above may be contemplated particularly when made in combination with substitution(s) made within a listed peptide. For example a change may be contemplated to restore structure or biological activity of the variant molecule. Such compensatory changes and changes to include deletion or addition of particular ammo acid residues from the FVIII polypeptide resulting in a variant with desired activity and in combination with changes in any of the disclosed peptides fall under the scope of the present.

In as far as this invention relates to modified FVIII, compositions containing such modified FVIII proteins or fragments of modified FVIII proteins and related compositions should be considered within the scope of the invention. A pertinent example in this respect could be development of peptide mediated tolerance induction strategies wherein one or more of the disclosed peptides is administered to a patient with immunotherapeutic intent. Accordingly, synthetic peptides molecules, for example one of more of those listed in TABLE 1, are considered embodiments of the invention. In another aspect, the present invention relates to nucleic acids encoding modified FVIII entities. In a further aspect the present invention relates to methods for therapeutic treatment of humans using the modified FVIII proteins. In a further aspect still, the invention relates to methods for therapeutic treatment using pharmaceutical preparations comprising peptide or derivative molecules with sequence identity or part identity with the sequences herein disclosed.

The invention will now be illustrated, but not limited by the following examples. The foregoing text and examples below refer to the following figures:
**FIGURE 1** provides a list of peptide sequences in human Factor VIII with potential human MHC class II binding activity Peptides are 13-mers, amino acids are identified using single letter code.
**FIGURE 2** depicts the scheme for pooled peptide screening. In the present study peptides were 15mers and overlapped each successive peptide by 12 residues.

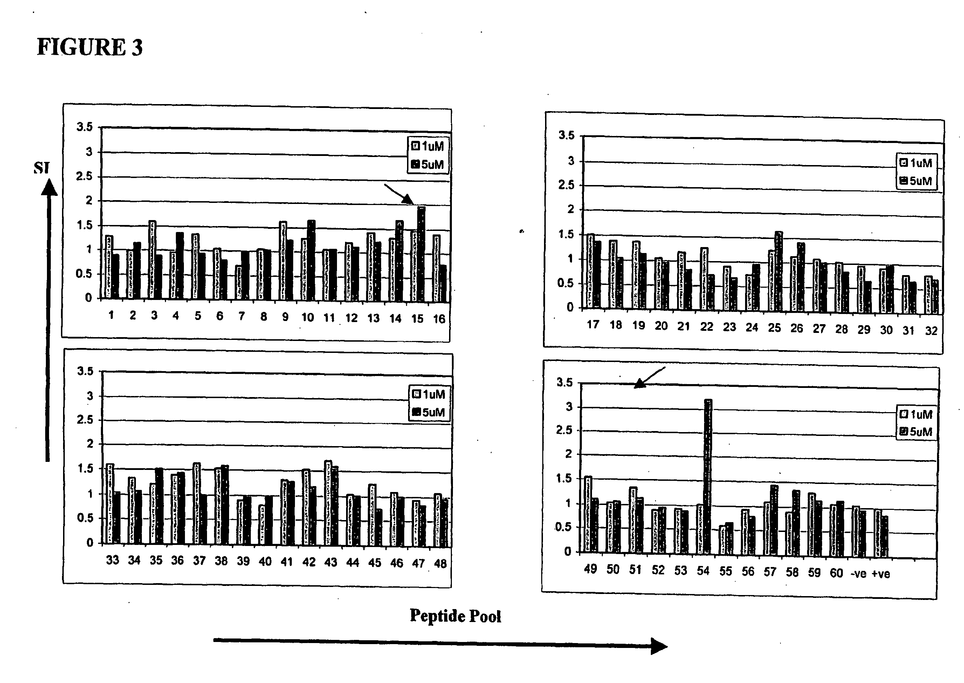

**FIGURE 3** provides histograms depicting results of *in vitro* T-cell proliferation assays in response to treatment with FVIII derived synthetic peptides. In this example a FVIII T-cell line isolated from a haemophiliac donor was used to screen overlapping peptide pools spanning the FVIII sequence. Arrows indicate pools that induce T cell proliferation and which were subsequently decoded.

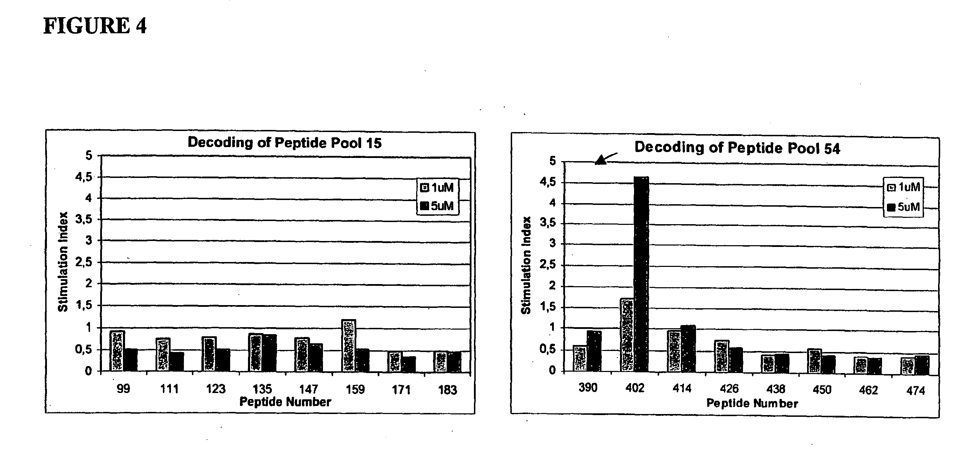

**FIGURE 4** provides histograms depicting results of *in vitro* T-cell proliferation assays in response to treatment with FVIII derived synthetic peptides. In this example a T-cell line specific for FVIII isolated from a haemophiliac donor was used to decode two peptide pools that induce T-cell proliferation. Peptides containing T-cell epitopes are indicated with an arrow.

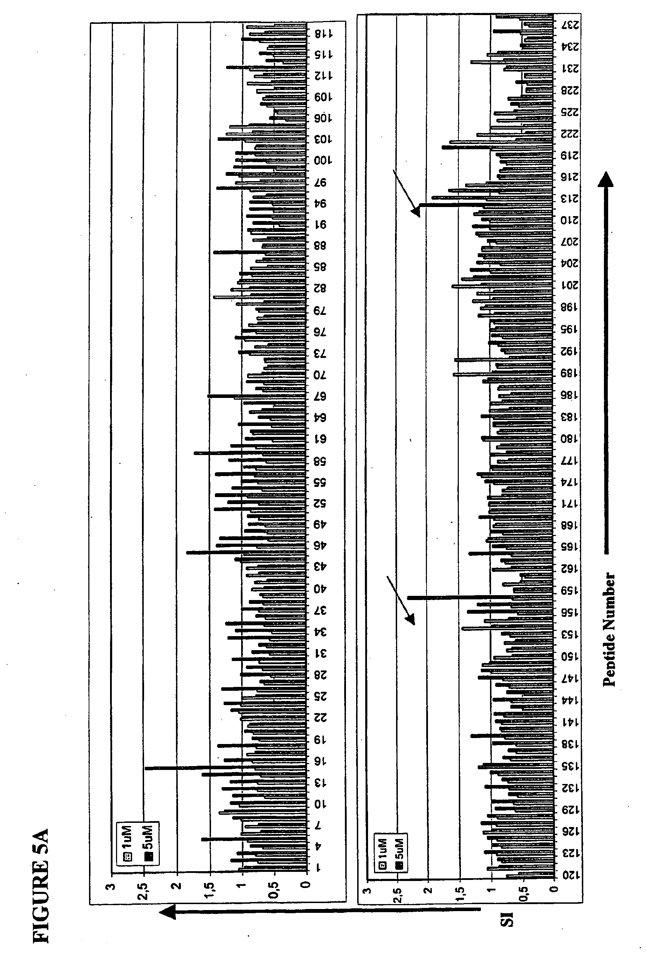

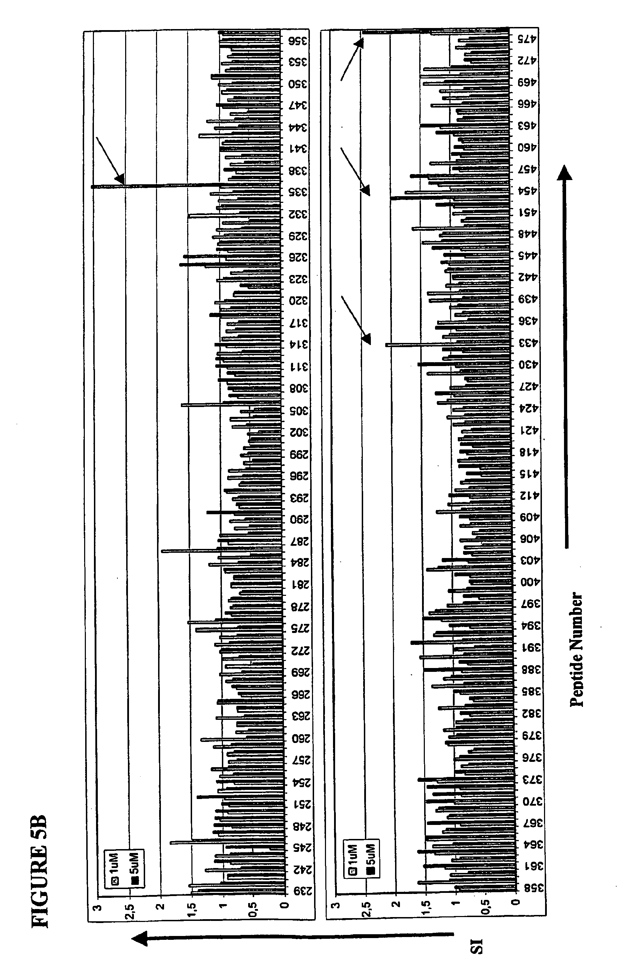

**FIGURE 5** provides histograms depicting results of *in vitro* T-cell proliferation assays in response to treatment with FVIII derived synthetic peptides. In this example cells from two donors were used to screen peptides spanning the FVIII sequence. Both donor #1 (A) and donor #2 (B) had identical HLA-DR allotypes (DRB 1*03, DRB1*04, DR3 and DR4*01). Arrows indicate peptides that contain T cell epitopes.

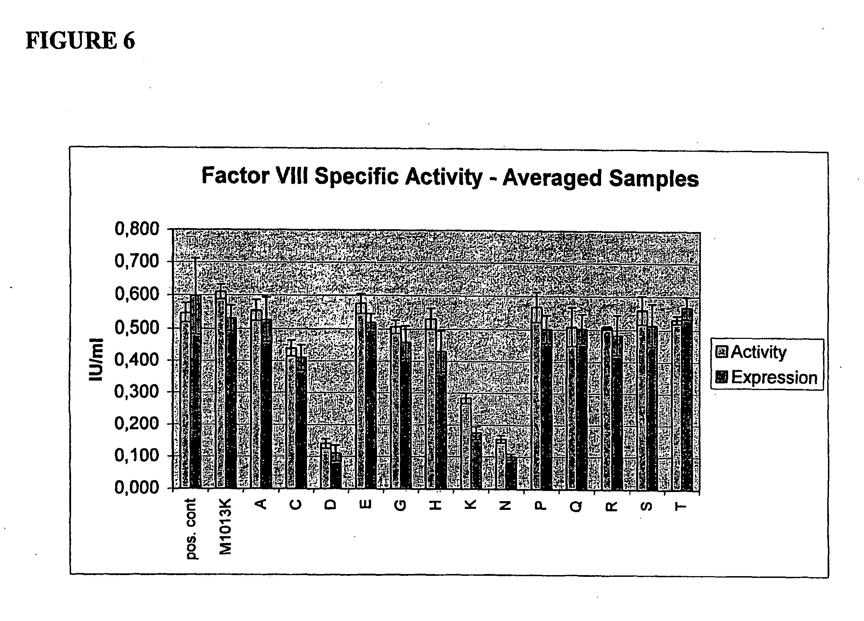

**FIGURE 6** provides a graph showing activity data and protein expression levels for mutants ofpeptide 8 (P8): Positive control is wt factor VIII. M1013K depicts expression and activity data for a FVIII molecule containing the M10103K substitution. Remaining columns are FVIII molecules combining the M1013K substitution with additional changes at I1011 to the indicated amino-acid.

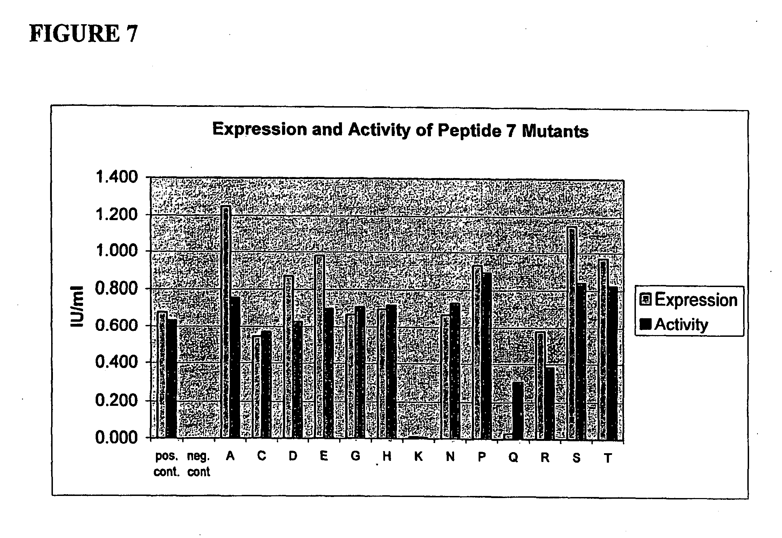

**FIGURE 7** provides a graph showing activity data and protein expression levels for mutants ofpeptide 7 (P7): Positive control is wt factor VIII, negative control is no DNA in transfection. Remaining samples are V823 mutants with the amino-acid change as indicated.

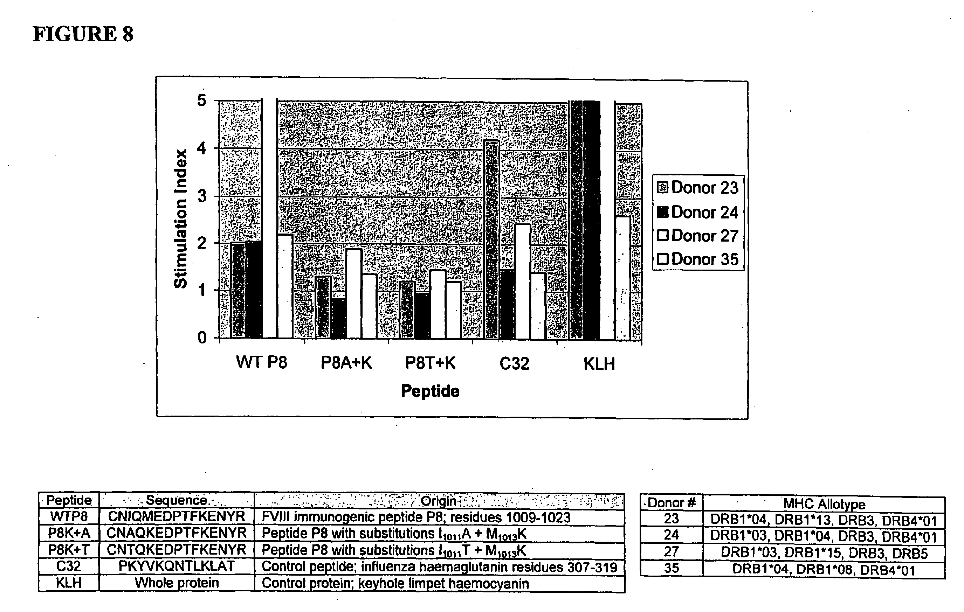

**FIGURE 8** provides exemplary T-cell assay data showing mutant peptides with a stimulation index of < 2,0 under conditions whereby the wt sequence peptide shows a stimulation index >2.0. Peptide sequences and PBMC donor allotype data are tabulated.
**FIGURE 9** shows a representation of the results achieved using the software simulation of peptide MHC class n binding, Results are shown for 18 different HLA-DR allotypes, each vertical column indicates the binding for a single allotype. Panel A shows the binding profile detected for the wt FVIII sequence around peptide 7. The peptide 7 sequence is highlighted. Panel B shows the binding profile detected for a modified peptide 7 sequence containing the substitution V₈₂₃A and demonstrates loss of a high affinity ligand for multiple allotypes. In each panel the predicted MHC binding is depicted by denoting the first residue of each 13mer MHC class II ligand. The intensity of binding is denoted H, M or L based on the calculated binding score for each ligand for each allotype as indicated. Physical binding studies have previously indicated that scores of <500,000 constitute a negligible binding interaction.

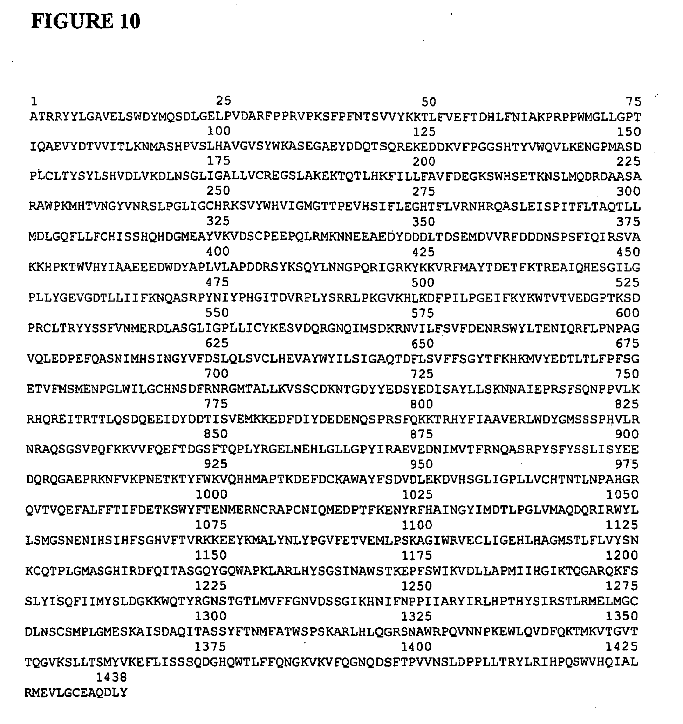

**FIGURE 10** shows the amino acid sequence and numbering for a wild-type (WT) B-domain deleted human FVIII sequence. Amino acids are depicted using single letter code.

### EXAMPLE 1

### Method for establishment of T cell lines and clones.

Peripheral blood mononuclear cells (PBMC) were isolated from blood obtained from haemophiliac patients, and cryogenically stored under liquid nitrogen.
Blood samples were provided with fully informed consent and working under local ethical approval of the Addenbrooke's Health Care Trust.
T cell lines were established by stimulating antigen specific T cells in bulk cultures using FVIII followed by several cycles of IL-2 induced expansion. Initially PBMC were incubated (at 37°C in a humidified atmosphere of 5% CO₂) at 2x10⁶ in 2ml AIM V media containing 4ug/ml FVIII (Refacto^{™}) in 24 well plates. After 7 days incubation 100U/ml. IL-2 was added and cultures were incubated for a further 3 days. T blasts were collected and counted upon completion of the 10 day antigen/lL-2 stimulation, In order to retain antigen specificity T blasts were subjected to a second round of antigen stimulation using γ-irradiated autologous PBMC as antigen presenting cells. This was achieved by incubating 1x10⁶ autologous PBMC/well in a 24 well plate with 4µg/ml FVIII for 1 hour in 0.75ml AIM V (containing 5% heat inactivated human AB serum) before being subjected to 4000 rads γ-irradiation. Autologous T blasts were added in 0.25ml AIM V at 4x10⁵ cells/ml to the γ-irraditated antigen presenting cells (pre-loaded with FVIII) and incubated for 3 days. T blasts were expanded by stimulating cells with 100U/ml IL-2 for 3 days; cultures were then supplied with fresh IL-2 (final concentration of 100U/ml) at 3 day intervals for a total of 9 days. To ensure that all expanded T blasts were antigen specific a third round of antigen stimulation was performed, where T blasts were collected and resuspended at 4x10⁵cells/ml in AIM V media. As described before antigen presenting cells were generated by incubating 1x10⁶ γ-irradiated autologous PBMC in a 24 well plate with 4ug/ml FVIII for 1 hour in 0.75ml AIM V (containing 5% heat inactivated human AB serum). Autologous T blasts in 0.25ml AIM V at 4x14⁵ cells/ml were added to the γ-irradiated antigen presenting cells and incubated for 3 days. A final expansion in 10U/ml. IL-2 was performed 3 days before T blasts were collected and used to screen peptide pools.

### Cloning from Bulk Cultures

After the third stimulation with FVIII antigen T blasts were collected and resuspended by serial dilution to a density of 4x10²-1x10⁴ cells/ml (2 x final culture density). Autologous PBMC were thawed and resuspended to 2x10⁶ cells/ml (2 x final culture density) in a polyproplene tube. PBMC were then exposed to 4000 rads γ-irradiation and were used as antigen presenting cells to select antigen reactive T cell clones by limiting dilution. γ-irradiated antigen presenting cells (1x10⁶ final density) were mixed with the T blasts (2x10²-5x10³ final density), 1-10ug/ml FVIII antigen and 100U/ml IL-2. T cell clones were established in Terasaki plates by adding 20µl of the APC, T blast, FVIII and IL-2 mixture to each well. Limiting dilution cloning was performed using 2-50 T blasts/well of a Terasaki plate.

### Selection and Maintenance of T Cell Clones

T blasts were incubated with FVIII antigen, IL-2 and γ-irradiated autologous antigen presenting cells for approximately 14 days. After identifying wells that contained cells showing unequivocal growth, T blasts were transferred to a single well of a round bottom 96 well plate containing 1x10⁵ γ-irradiated allogenic PBMC, 100U/ml IL-2 and 1µg/ml phytohaemaglutinin (PHA) in a final volume 200µl AIM V (with 1% heat inactivated human AB serum). T cell clones were split when cells became confluent, and ultimately transferred to a single well of 24 well plate containing 1x10⁶ γ-irradiated allogenic PBMC (feeder cells), 100U/ml IL-2 and 1µg/ml phytohaemaglutinin (PHA) in a final volume of 2ml AIM V (with 1% heat inactivated human AB serum). Routine maintenance of T cell clones involved stimulation with fresh PHA and allogenic feeder cells every 2-3 weeks (depending on cell growth) and twice weekly stimulation with 100U/ml IL-2- Only T cell clones that proved to be FVIII specific were expanded and used to screen FVIII peptides.

### EBV Transformation of Autologous B Cells.

B cells from PBMC preparations were immortalized to generate B lymphoblastoid cell lines (BLCL) by adding 3ml of filtered (0.45µ) B95.8 supernatant to 4x10⁶PBMC and incubating at 37°C for 1 hour. PBMC were pelleted and resuspended in 2ml RPMI containing 5% heat-inactive foetal calf serum (PCS) and 1 µg/ml cyclosporin A. After 7 days incubation 1ml of culture media was replaced with fresh RPMI containing 5% FCS and 2ug/ml cyclosporin A (to give a final concentration of 1µg/ml cyclosporin A), This feeding regime was repeated on days 14 and 21 after which cells were split when necessary using RPMI containing 5% FCS and expanded into tissue culture flasks.

### Screening FVIII Peptides Using T Cell Lines/Clones

Peptides of 15 residues in length and overlapping with the previous peptide by increments of 12 amino acids were synthesized (Pepscan, Netherlands). Peptides were initially solubilized at 10mM in 100% dimethylsulphoxide (DMSO) for storage. Peptide pools were generated to simultaneously screen a large number ofpeptides against FVIII specific T cell lines. Pools were organized such that each pool contained overlapping peptides of subsequent pools by using this approach T cell epitopes that overlap two peptides will result in inducing proliferation two separate pools. Each pool typically consisted of 8 peptides with each peptide being tested at either 1 or 5µM. The peptide pool strategy is illustrated in FIGURE 2.
Autologous PBMC (for T cell lines) or EBV transformed BLCL (for T cell clones) were used as antigen presenting cells by resuspending 1x10⁵ PBMC or BLCL in 50µl AIM V media which was then added to each well of a round bottom 96 well plate. Peptide pools were added in triplicate wells for each pool at both concentrations (1 or 5µM. Antigen presenting cells and peptide pools were incubated for 1 hour at 37°C before exposure to 4000 rads γ-irradiation. BLCL were pretreated with 1µg/ml Mitomycin C for 1 hour at 37°C followed by washing 4 times in AIM V when used as antigen presenting cells (instead of γ-irradiated autologous PBMC) for T cell clones. Antigen specific T cell lines or T cell clones were then added at 5x10⁴ cells per well and the cultures were incubated for 3 days. On the third day each well was pulsed with 1 µCi [³H]-Thymidine for a minimum of 8 hours. After harvesting the plates onto filtermats the cpm/well was determined using a Wallac Microplate Beta counter. FIGURE 3 shows an epitope map generated using a T cell line, where T blasts were used to identify peptide pools containing T cell epitopes, those pools were then decoded to identify the individual peptide containing the T cell epitope.

### Naïve T Cell Epitope Map using PBMC from Healthy Donors

Blood from 40 healthy HLA-DR typed donors was used to isolate PBMC which were used to screen individual FVIII peptides at two concentrations (1 and 5µM). Since there were insufficient numbers of PBMC from each donor to screen all FVIII peptides, donors were split into two groups where the first 20 donors were used to screen peptides spanning the first half of the molecule and the second set of donors used to screen the retaining peptides. Donors were selected according to MHC class II allotypes expressed in order to cover a large number of allotypes present in the world population. MHC allotypes were detected using

The tissue types for all PBMC samples were assayed using a commercially available reagent system (Dynal, Wirral, UK), Assays were conducted in accordance with the suppliers recommended protocols and standard ancillary reagents and agarose electrophoresis systems.

PBMC contain physiological numbers of naïve T cells and antigen presenting cells. These cells were used at a density of 2x10⁵ cells/well (96 flat bottom plate) to screen peptides at 1 and 5µM in triplicate 200µl cultures. Cells were incubated with peptides at 37°C for 6 days before pulsing each well with 1µCi [³H]-Thymidine for a minimum of 8 hours. Cultures were harvested onto filtermats and the cpm/well was determined using a Wallac Microplate Beta counter. FIGURE 5 shows an epitope map of FVIII generated using two donors to screen separate halves of the molecule.

### EXAMPLE 2

### Method for cloning and expression of Factor VIII

### mRMA Extraction and cDNA Synthesis:

Human liver tissue was obtained from a hospital pathology department. The sample was diced and dispensed as 50mg aliquots and stored in liquid nitrogen. One 50mg aliquot was homogenised and mRNA extracted directly using a PolyATract System 1000 kit (Promega) according to the manufacturers instruction, yielding approx. 10µg mRNA. lug aliquots of mRNA were reverse transcribed in 20µl reactions using the ImProm-II reverse transcription system (Promega) using an oligo(dT)₁₅ primer according to the manufacturers instructions. The reverse transcriptase enzyme was then inactivated by heating to 70°C for 15mins.

### Polymerase Chain Reaction Amplification of Factor VIII sequences:

Since a B domain deleted variant of factor VIII was to be cloned, the cDNA was amplified from the cDNA in two halves. The 5' end of the mRNA was amplified using the following primers:
SEQ ID NO. 1 GCATCGCGCGCTAGCAATAAGTCATGCAAATAGAG
SEQ ID NO. 2 GAAGCTCCTAGGTTCAATGGCATTGTTTTACTCA

Seq ID No. 1 contains two restriction enzyme sites to facilitate cloning plus 24 nucleotides of the factor VIII mRNA sequence surrounding the ATG start codon (bold). Seq ID No. 2 is complimentary to nucleotides 2420 to 2454 of the factor VIII mRNA and contains two changed nucleotides at positions 2445 and 2448 (shown in bold) which introduce a new restriction enzyme site whilst leaving the protein sequence unaffected.

The 3' end of the factor VIII gene was amplified using the following primer pair:
SEQ ID NO.3:
   TGAGTCTTAAGCTAGCTAGATACCTAGGAGCTTCTCCCAAAACCCACCAGTCTTGAAACGCC
SEQ ID NO. 4:
   TACGTCTCGAGTCAGTAGAGGTCCTGTGCCTCGCA

Seq ID No. 3 contains restriction enzyme site NheI to facilitate cloning plus nucleotides 2442 to 2457 of the factor VIII mRNA, fused to nucleotides 5140 to 5164. This primer therefore creates the junction between the heavy and light chains where almost the entire B domain is absent This primer also contains the nucleotide changes at positions 2445 and 2448 (shown in bold) which create the new restriction enzyme site also found in Seq ID No, 2. Seq ID No. 4 contains the final 24 nucleotides of the factor VIII coding sequence plus a restriction enyme site to facilitate cloning.
PCR reactions were done using Expand HiFi polymerase (Roche) using the supplied buffer containing MgCl₂ in a final volume of 50µl. The reactions were made up to 1x buffer containing 200µM of each dNTP, 50pmols of each primer (either seq ID No. 1 plus No.2 or seq ID No. 3 plus No. 4), 2.5units RnaseH, and 5µl of reverse transcriptase reaction mix. The reactions were incubated at 37°C for 30min. in order for the RNA in the RNA/cDNA hybrid to be degraded by the RnaseH in order to increase the efficiency of the PCR reaction. The reactions were then heated to 94°C for 2min. for initial denaturation, followed by cycling for the following temperatures and times: 94°C 30sec., 55°C 30sec., 72°C 2.5mm. During the first annealing step the recommended amount of polymerase was added and the reaction cycled 20 times. During the annealing step following the 20^{th} extension step, a second aliquot of polymerase was added and the reaction cycled a further 20 times. The reaction was then incubated at 72°C for 10min to ensure complete polymerisation of all strands.

### Closing and Assembly of the Factor VIII Gene:

One half of the PCR reactions were separated on a 1% agarose gel and the 2286bp band corresponding to the 5' end of the factor VIII cDNA and the 2015bp band corresponding to the 3' end were excised and purified from the agarose via a Qiagen Crel Extract kit.
The PCR products were then ligated into a PCR product cloning vector (pGEM-T Easy Vector System, Promega) as instructed by the manufacturers. 1 µl of each ligation reaction was electroporated into 20µl electrocompetent *E. coli* strain XL1-Blue (Stratagene) as recommended by the supplier using a 0.1cm gap cuvette. The cells were resuspended and allowed to recover also as recommended by the supplier. 10µl and 100µl aliquots of the electroporated cells were plated out on LB agar plates containing 100µg/ml ampicillin, 80µg/ml Xgal and 0.5mM IPTG and grown at 37°C overnight.

White colonies were picked and dispersed in 20µl water. A sample from each resuspended colony was analysed via PCR in a reaction volume of 20µl using Taq polymerase (Roche) with the supplied buffer. Reactions were made up in 1x buffer containing 200µM of each dNTP, 50pmols each primer being the standard M13 forward and reverse primers (SEQ ID Nos 5 and 6 herein), and 1µl resuspended colony. The reactions were then heated to 94°C for 2min. for initial denaturation, followed by cycling x20 for the following temperatures and times: 94°C 30sec., 60°C 30sec., 72°C 2min. The reaction was then incubated at 72°C for 10min to ensure complete polymerisation of all strands.

A 5µl sample of each reaction was separated on a 1% agarose gel. Samples containing bands at approx, 2300bp were deemed positive for the factor VIII 5' half insert and those containing bands at approx. 2150bp were deemed positive for the factor VIII 3' half insert. For each insert, eight colonies were each inoculated into 5ml liquid cultures of 2YT broth/100ug/ml ampicillin and grown overnight at 37°C with shaking. Plasmid was prepared from 1.5ml of each culture using a Qiagen mini-prep kit and the plasmids were sent to a contract sequencing facility for sequence determination using the following primers:

| 5' half clones: | | |
|---|---|---|
| SEQ ID No. 5 | CGCCAGGGTTTTCCCAGTCACGAC | (M13 forward) |
| SEQ ID No. 6 | AGCGGATAACAATTTCACACAGGA | (M13 reverse) |
| SEQ ID No. 7 | ATGATCAGACCAGTCAAAGG | (factor VIII mRNA nt573-592) |
| SEQ ID No. 8 | CAGGAAATCAGTCTATTGGC | (factor VIII mBNA ut975-994) |
| SEQ ID No. 9 | TGGGTACATTACATTGCTGC | (factor VIII mRNA nt1372-1391) |
| SEQ ID No. 10 | ACAGTGACTGTAGAAGATGG | (factor VIII mRNA nt1768-1787) |
| SEQ ID No. 11 | GTCTTCTTCTCTGGATATACC | (factor VIII mRNA nt2179-2199) |

| 3' half clones: | | |
|---|---|---|
| SEQ ID No. 5 | CGCCAGGGTTTTCCCAGTCACGAC | (M13 forward) |
| SEQ ID No. 6 | AGCGGATAACAATTTCACACAGGA | (M13 reverse) |
| SEQ ID) No. 12 | GAGTAGCTCCCCACATGTTC | (factor VIII mRNA ut5370-5361) |
| SEQ ID No. 13 | GTGCACTCAGGCCTGATTGG | (factor VIII mRNA nt5786-5767) |
| SEQ ID No. 14 | AGGTGTTTTTGAGACAGTGG | (factor VIII mRNA nt6187-6168) |
| SEQ ID No. 15 | GAGGAAATTCCACTGCAACC | (factor VIII mRNA nt6594-6575) |
| SEQ ID No, 16 | AATCTCTGCTTACCAGCATG | (factor VIII mRNA nt6993-6974) |

Plasmid pCF85 was found to code for the correct amino-acid sequence for the 5' half of the factor VIII gene. Plasmid pCF83 was found to code for the correct amino-acid sequence for the 3' half of the factor VIII gene,

pCF83 was digested with Bst98I and XhoI and the released 2.0Kbp fragment containing the 3' half of the factor VIII gene was purified via agarose gel electrophoresis and cloned into similarly cut and purified pLitmus (NEB) using standard techniques. Positive bacterial colonies were identified via PCR as described above and one clone, pCLF83, selected, grown and DNA prepared.

pCF85 was digested with BssHII and the ends made flush with T4 DNA polymerase (NEB) in the presence of 100µM each dNTP. The reaction was then heated to 70°C for 10min and then digested with AvrII. The released 2.3kbp fragment was purified via agarose gel electrophoresis and cloned into pCLF83 which had been digested with Bst981, flush ended as above, digested with AvrII and gel purified. Positive bacterial colonies were identified via PCR screening as above using primers Seq ID No. 11 and Seq ID No. 17 (ATCAGTAAATTCCTGGAAAAC factor VIII mRNA nt5448-5428]). These primers amplify a fragment across the junction between the two halves of the factor VIII gene and therefore a product of approx. 570bp is seen only if the cloning has been successful. A positive colony was selected and termed pCLF8. This colony was grown and DNA prepared and sequenced. Correct junctional sequences were confirmed using primers Seq ID No. 6 and Seq ID No. 5. The junction between the 5' and 3' halves was also verified using primer Seq ID No.17

### Expression and Activity Analysis of Factor VIII:

The FVIII protein was expressed using a modified variant of the vector pCI (Promega, Southampton, UK). The unmodified pCI vector is 4.0kbp long and contains a CMV enhancer/promoter and SV40 late polyadenylation signal for mammalian expression and contains sequences necessary for bacterial propogation. The vector also contains a bacteriophage F1 origin and this was removed by digesting the plasmid with restriction enzymes BamHI and EcoO109I. The digest products were blunt ended using T4 DNA polymerase as described above, followed by separation through a 1% agarose gel, The 3.2kbp vector fragment was purified from the gel, self-ligated and transformed into bacterial strain XL1-blue as described above. Colonizes were picked, grown overnight and plasmids mini-prepped as described above. Samples of the purified plasmids were digested with restriction enzyme NgoMIV, which is present in the F1 origin sequence, and analysed via agarose gel electrophoresis. A resistant plasmid was selected and termed pCIΔ.
pCIΔ was digested with restriction enzymes NheI and XhoI and the vector fragment purified via agarose gel electrophoresis. pCLF8 was similarly digested and the 4.4kbp fragment containing the factor VIII gene was purified via agarose gel electrophoresis and cloned into the cut pCIΔ using standard techniques. Positive bacterial colonies were identified via PCR analysis using primers Seq ID No.1 and Seq ID No. 17 as described above.
One positive colony, termed pCIF8 was selected and inoculated into 50ml 2YT broth containing 100µg/ml ampicillin and grown at 37°C overnight with vigorous shaking. Highly pure plasmid DNA was prepared using a Qiagen midi-prep kit. Plasmid DNA was quantified spectrophotometrically, diluted to 0.2µg/µl and filter sterilised through a 0.2µm pore 1.5cm diameter filter unit (Nalgene).
HEK 293 cells (ATCC CRL-1573) were maintained in continuous culture in 75cm² tissue culture flasks in DMEM containing Glutamax-I, sodium pyruvate and 4500mg/ml glucase (Invitrogen cat. no. 31966-021), supplemented with 10% heat inactivated foetal bovine serum (Perbio cat. no. CH30160.03). Cells were grown at 37°C/5% CO₂ and subcultured by diluting 1/5 every 48h. HEK 293 cells adhere only weakly to tissue-culture plastic and can be detached by washing the flask surface.
HEK293 cells were transfected in 24 well poly-L-lysine coated tissue culture dishes (Beckton Dickinson) using Lipofectamine 2000 (Invitrogen) as described by the manufacturer. Almost confluent cells in a 75cm tissue culture flask were washed with phosphate buffered saline (PBS) and detached from the tissue culture flask using trypsin/EDTA solution. The trypsin was inactivated by addition of an equal volume of growth media. Cells were counted in a haemocytometer and the cell suspension centrifuged at 1200rpm for 5min. The supernatant was removed and the cell pellet resuspended in growth media at a density of 4x10⁵ cells/ml. 0.5ml of the cell suspension was dispensed into each well of a 24 well tissue culture dish and incubated overnight at 37°C/5% CO₂. Prior to transfection the media was removed from each well and replaced with 0.5ml fresh media. 0.8µg plasmid pCIF8 and negative control plasmid pCIΔ were each diluted to 50µl in Optimem (Invitrogen cat. no. 51985-026). For each transfection, 2µl Lipofectamine 2000 was diluted to 50µl, incubated at room temperature for 5min and then combined with diluted plasmid followed by a further incubation at room temperature for 20min. Transfections were done in triplicate and each 100µl plasmid/lipid mixture was then added drop-wise to one well of the 24 well plate. The plates were then incubated at 37°C /5% CO₂. 10µl aliquots were removed from each transfection at 24h, 48h and 72h. Aliquots from each triplicate were combined to give a total volume of 30µl per sample and frozen at -80°C prior to activity analysis.
Factor VIII activity in the supernatant was assayed using a Coatest F8:C/4 kit (Chromogenix) in microtitre format according to the manufacturers instructions. Supernatant samples were thawed on ice, diluted 1/20 and 1/100 in assay buffer and assayed in duplicate. Standards were lyophilised plasma samples quantified against international standards for factor VIII activity (Chromogenix). One vial of plasma was reconstituted in 1ml water as described by the manufacturer. The accompanying data sheet was consulted for the level of factor VIII activity in the plasma which was then diluted to 100% (1IU/ml) by addition of assay buffer. The standards were then diluted as described in the manufacturers microtitre assay protocol. After 24h the supernatants from cells transfected with pCIF8 were found to contain 0.16 IU/ml activity which increased to 0,74 IU/ml at 48h and then dropped back to 0.5 IU/ml at 72h. No activity was seen in supernatants from cells transfected with control vector. Therefore factor VIII was successfully expressed in quantities sufficient for determining activity of mutants.

### Mutagenesis of Amino-Acids in Peptide P10 (TABLE 2)

Peptide 10 contains four hydrophobic residues which have the potential to be the primary anchor for interaction with MHC Class II molecules, F1207, I1208, I1209 & M1210 (numbering according to mature B domain deleted sequence). Therefore these four residues were targeted for mutation to residues other than those which can potentially be primary anchors. F1207 was changed to:A, H, K, N, Q and R; I1208 was changed to: A, T, D, N; I1209 was changed to: A, C, D, N, P; M1210 was changed to: A, K, N and Q. Mutagenesis was done via overlap PCR using established protocols well known to those skilled in the art. Peptide 10 lies within a fragment of the factor VIII nucleotide sequence bounded by PspOMI and SphI restriction sites. PCR primers for amplification of these fragment were synthesised corresponding to sequences just outside this region (Seq ID No. 18 and No. 19 below). For mutagenesis of F1207, internal primers were synthesised as described below:

| | | |
|---|---|---|
| SEQ ID No.18: | GGACACATTAGAGATTTTCA | (gene nt.3463-3482) |
| SEQ ID No.19: | CAGTAATCTGTGCATCTGAT | (gene nt.3949-3930) |
| SEQ ID No.20: | CTGAGAGATGTAGAGGCT | (gene nt.3675-3658) |
| SEQ ID No.21: | AGCCTCTACATCTCTCAGgccATCATCATGT | (F1207A) |
| SEQ ID No.22: | AGCCTCTACATCTCTCAGcacATCATCATGT | (F1207H) |
| SEQ ID No.23: | AGCCTCTACATCTCTCAGaagATCATCATGT | (F1207K) |
| SEQ ID No.24: | AGCCTCTACATCTCTCAGaacATCATCATGT | (F1207N) |
| SEQ ID No.25: | AGCCTCTACATCTCAGcagATCATCATGT | (F1207Q) |
| SEQ ID No.26: | AGCCTCTACATCTCTCAGcgcATCATCATGT | (F1207R) |

PCR were done using Expand HiFi polymerase (Roche) using the supplied buffer containing MgCl₂ in a final volume of 50µl. The 5' fragment reaction contained 1x buffer containing 200µM of each dNTP, 50pmols of each primer (Seq ID No. 18 plus No.20), 100ng pCIF8, and 2,5 units Expand polymerase. Six 3' fragment reactions were set up and contained 1x buffer containing 200µM of each dNTP, 50pmols of each primer (Seq ID No. 19 plus either Seq ID No.21, 22, 23, 24, 25 or 26), 100ng pCIF8 and 2.5units Expand polymerase. The reactions were then heated to 94°C for 2min, for initial denaturation, followed by cycling for the following temperatures and times: 94°C 30sec., 50°C 30sec., 72°C 30sec. The reactions were cycled 20 times and then incubated at 72°C for 10min to ensure complete polymerisation of all strands.
One half of each PCR was separated on a 1% agarose gel and the 5' fragment of 226bp and the six different 3' fragments of 292bp were excised from the gel, and purified using a Qiagen Gel Extract kit. The 5'fragment was then joined to each of the 3' fragments as follows: six further PCRs were done as described above using Expand HiFi polymerase using as template 2µl of eluted 5' fragment with 2µl of each of the six eluted 3' fragments with primers Seq ID No. 18 and Seq ID No. 19. Half of each of these reactions were separated on a 1% agarose gel and the six bands of 486bp were purified as described above. Each PCR product was digested with restriction enzymes PspOMI and SpbI in a total reaction volume of 50µl overnight at 37°C. 4µg plasmid pCIF8 was similarly digested for 2h at 37°C and half of the plasmid and PCR fragment digests were run through a 1% agarose gel. The vector band of 7.2kbp and the PCR fragments of 391bp were excised from the gel and purified as above into final volumes of 30µl each in water. 1 µl of vector was ligated to 3 µl of each of the six fragments in standard 10µl ligation reactions using T4 DNA ligase and supplied buffer (Invitrogen). The reactions were incubated at room temperature for 4h. 1µl of each ligation reaction was electroporated into 20µl electrocompetent *E*. *coli* strain XL1-Blue (Stratagene) as recommended by the supplier using a 0.1 cm gap cuvette. The cells were resuspended and allowed to recover also as recommended by the supplier. 10µl and 100µl aliquots of the electroporated cells were plated out on LB agar plates containing 100µg/ml ampicillin and grown at 37°C overnight.

Four colonies from each plated ligation were picked and grown up overnight in 5ml liquid culture in 2YT broth plus 100µg/ml ampicillin at 37°C with vigorous shaking. Plasmid DNA was prepared from 1.5ml of each culture using a Qiagen Mini-Prep kit. Samples of each plasmid were DNA sequenced using primer Seq ID No. 18. One plasmid from each group of four, with the correct sequence was selected and stored for analysis via transfection for activity and expression levels.
This same general procedure was followed for creating the mutations at I1208, I1209 and M1210. The primers used for each amino-acid were as follows:

| I1208: | | |
|---|---|---|
| SEQ ID No.18: | GGACACATTAGAGATTTTCA | (gene nt.3463-3482) |
| SEQ ID No.19: | CAGTAATCTGTGCATCTGAT | (gene nt.3949-3930) |
| SEQ ID No.20: | CTGAGAGATGTAGAGGCT | (gene nt.3675-3658) |
| SEQ ID No.27: | AGCCTCTACATCTCTCAGTTTgccATCATGT | (I1208A) |
| SEQ ID No.28: | AGCCTCTACATCTCTCAGTTTaccATCATGT | (I1208T) |
| SEQ ID No.29: | AGCCTCTACTCTCTCAGTTTgacATCATGT | (I1208D) |
| SEQ ID No.30: | AGCCTCTACATCTCTCAGTTTaacATCATGT | (I1208N) |

| I1209: | | |
|---|---|---|
| SEQ ID No.18: | GGACACATTAGAGATTTTCA | (gene nt.3463-3482) |
| SEQ ID No.19: | CAGTAATCTGTGCATCTGAT | (gene nt.3949-3930) |
| SEQ ID No.20: | CTGAGAGATGTAGAGGCT | (gene nt.3675-3658) |
| SEQ ID No.31: | AGCCTCTACATCTCTCAGTTTATGgccATGTATA | (I1209A) |
| SEQ ID No.32: | AGCCTCTACATCTCTCAGTTTATCtgcATGTATA | (I1209C) |
| SEQ ID No.33: | AGCCTCTACATCTCTCAGTTTATCgacATGTATA | (I1209D) |
| SEQ ID No.34: | AGCCTCTACATCTCTCAGTTTATCaacATGTATA | (I1209N) |
| SEQ ID No.35: | AGCCTCTACATCTCTCAGTTTATCcccATGTATA | (I1209P) |

| M1210: | | |
|---|---|---|
| SEQ ID No.18: | GGACACATTAGAGATTTTCA | (gene nt.3463-3482) |
| SEQ ID No.19: | CAGTAATCTGTGCATCTGAT | (gene nt.3949-3930) |
| SEQ ID No.20: | CTGAGAGATGTAGAGGCT | (gene nt.3675-3658) |
| SEQ ID No.36: | AGCCTCTACATCTCTCCAGTTTATCATCgccTATAGTC | (M1210A) |
| SEQ ID No.37: | AGCCTCTACATCTCTCAGTTTATCATCaagTATAGTC | (M1210K) |
| SEQ ID No.38: | AGCCTCTACATCTCTCAGTTTATCATCaacTATAGTC | (M1210N) |
| SEQ ID No.39: | AGCCTCTACATCTCTCAGTTTATCATCcagTATAGTC | (M1210Q) |

Clones for each mutation which had been verified by sequence analysis were transfected into HEK 293 cells in 24 well poly-L-lysine plates in duplicate as described above. Transfections were incubated for 48h at37°C/5% CO₂. Duplicate supernatants for each transfection were pooled and assayed for factor VIII activity as described above. Supernatants were also assayed for factor VIII expression levels using a paired anti-factor VIII antibody ELISA kit (Affinity Biologicals). This assay was modified to effectively quantify tissue culture supernatant material and was performed as follows: Capture antibody was diluted 1/100 in sodium carbonate/bicarbonate buffer pH9.6 and added 100µl per well to a Dynex Immulon 4 96 well ELISA plate. The plate was incubated overnight at 4°C. The wells were washed x4 with 100µl each of wash buffer (Tris buffered saline [TBS: 25mM Tris, 137mM NaCl, 3mM KCl, pH7.4 @ 25°C] containing 0.1 % Tween 20) and duplicate 100µl aliquots of diluted standards and tissue culture supernatants added per well. Standards were lyophilised plasma samples quantified against international standards for factor VIII activity (Chromogenix). One vial of plasma was reconstituted in 1ml water as described by the manufacturer. The accompanying data sheet was consulted for the level of factor VIII activity in the plasma which was then diluted to 100% (1IU/ml) by addition of sample dilution buffer (TBS containing 1% w/v bovine serum albumin). This was then diluted 1 in 4 with sample dilution buffer to give the 100% ELISA standard. This was then diluted further with sample dilution buffer to give standards representing 75%, 50%, 25% and 5%. Tissue culture supernatants were diluted in 4 with sample dilution buffer. The plate was incubated for 2h at room temperature and washed x4 with wash buffer, 100µl per well. 100µl horse radish peroxidase conjugate antibody was added per well and the plate incubated at room temperature for 1h. The plate was washed as before and 100µl of substrate (ready prepared TMB/peroxide solution: Sigma cat.no. T0440) added per well. The plate was incubated at room temperature for 15min followed by the addition of stop solution (2M sulphuric acid). The plate was read in an Anthos HTII plate reader using a 450nm filter.

Comparison of the activity values and expression levels revealed that all changes to F1207 resulted in absence of expression and hence activity. Mutation of I1208 to A resulted in expression and activity similar to unmodified factor VIII wheras changes to T and N resulted in 25% and 50% losses in activity. For I1209, only mutation to C allowed expression and the activity of this variant was similar to unmodified factor VIII. All changes to M1210 resulted in expression of proteins with appreciable activity with K and N being indistinguishable from unmodified factor VIII. Therefore I1208A, I1209C, M1210K and M1210N are useful mutations for the removal of the T cell epitope within peptide 10.

### Mutagenesis of Amino-Acids in Peptide P8 (TABLE 2)

Peptide 8 contains two amino-acids which are potential primary anchors for binding to MHC Class II molecules; I1011 and M1013 (numbering according to mature B domain deleted sequence). Therefore these two residues were targeted for mutation to residues other than those which can potentially be primary anchors. Comparison to the factor VIII sequences of other species (dog, mouse and rat) revealed that in dog factor VIII, M1013 is K. K was also shown to be the best replacement for M1210 for mutagenesis of peptide 10. Therefore M1013 was mutated to K alone and in combination with all amino-acids at I1011 which do not have the potential to form primary anchors (i.e. A, C, D, E, K, N, P, Q, R, S, T).

Mutagenesis was done via overlap PCR using established protocols well known to those skilled in the art. Peptide 8 lies within 490bp fragment of the factor VIII nucleotide sequence bounded by PflM1 and PspOMI restriction sites. PCR primers for amplification of this fragment were synthesised corresponding to sequences just outside this region (Seq ID No. 40 and No. 41 bellow). For mutagenesis of M1013K plus I1011X, internal primers were synthesised and used as described below:

| | | |
|---|---|---|
| SEQ ID No.40: | ATGAGACCAAAAGCTGGT | (gene nt.3026-3043) |
| SEQ ID No.41: | AGGCATTGATTGATCCG | (gene nt.3559-3543) |
| SEQ ID No.42: | ATTGCAGGGAGCCCTGCAGT | (gene nt.3087-3068) |
| SEQ ID No.43: | ACTGCAGGGCTCCCTGCAAT**AT**CCAG**aag**GAAGA | (MI013K) |
| SEQ ID No.44: | ACTGCAGGGCTCCCTGCAATgccCAGaagGAAGA | (I1011A, M1013K) |
| SEQ ID No.45 : | ACTGCAGGGCTCCCTGCAATtgcCAGaagGAAGA | (I1011C, M1013K) |
| SEQ ID No.46: | ACTGCAGGGCTCCCTGCAATgacCAGaagGAAGA | (I1011D, M1013K) |
| SEQ ID No.47: | ACTGCAGGGCTCCCTGCAATgagCAGaagGAAGA | (I1011E, M1013K) |
| SEQ ID No.48: | ACTGCAGGGCTCCCTGCAATggcCAGaagGAAGA | (I1011G, M1013K) |
| SEQ ID No.49: | ACTGCAGGGCTCCCTGCAATcacCAGaagGAAGA | (I1011H, M1013K) |
| SEQ ID No.50: | ACTGCAGGGCTCCCTGCAATaagCAGaagGAAGA | (I1011K, M1013K) |
| SEQ ID No.51: | ACTGCAGGGCTCCCTGCAATaacCAGaagGAAGA | (I1011N, M1013K) |
| SEQ ID No.52: | ACTGCAGGGCTCCCTGCAATcccCAGaagGAAGA | (I1011P, M1013K) |
| SEQ ID No.53: | ACTGCAGGGCTCCCTGCAATcagCAGaagGAAGA | (I1011Q, M1013K) |
| SEQ ID No.54: | ACTGCAGGGCTCCCTGCAATcgcCAGaagGAAGA | (I1011R, M1013K) |
| SEQ ID No.55: | ACTGCAGGGCTCCCTGCAATagcCAGaagGAAGA | (I1011S, M1013K) |
| SEQ ID No.56: | ACTGCAGGGCTCCCTGCAATaccCAGaagGAAGA | (I1011T, M1013K) |

The mutagenesis was done using the same general procedure described for mutagenesis of peptide 10 above, except that the 5' fragment was 62bp in length and the 3'fragment 492bp. The joned fragment was 534bp in length and was digested with PspOMI and PfIMI and cloned into similarly digested pCIF8.

One clone with the correct sequence was selected for each mutant and their activities and expression levels analysed as described above except that transfection were done in triplicate and each supernatant was analysed individually so that variations in expression/activity could be assessed.
Comparison of expression levels and activity for the above mutants revealed that M1013K alone was very similar to unmodified factor VIII. In addition, combination mutants containing M1013K plus I1011A, E, P, S or T were also indistinguishable from unmodified factor VIII. Therefore these mutations are useful for the removal of T cell epitopes associated with peptide 8.

### Mutagenesis of Amino-Acids in Peptide P7 (TABLE 2)

Peptide 7 contains one amino-acid which is a potential primary anchor for binding to MHC Class II molecules, V823 (numbering according to mature B domain deleted sequence). Therefore this residue was targeted for mutation to residues other than those which can potentially be primary anchors (i.e. A, C, D, E, K, N, P, Q, R, S, T).

Mutagenesis was done via overlap PCR using established protocols as previously. Peptide 7 lies within 766bp fragment of the factor VIII nucleotide sequence bounded by restriction enzymes AvrII and PflM1. PCR primers for amplification of this fragment were synthesised corresponding to sequences just outside this region (Seq ID No. 57 and No. 58 below). For Mutagenesis of V823X, internal primers were synthesised and used as described below:

| | | |
|---|---|---|
| SEQ ID No.57: | CGAGGACAGTTATGAAG | (gone nt.2214-2230) |
| SEQ ID No.58: | AGTGGGATCTTCCATCTG | (gene nt.3108-3091) |
| SEQ ID No.59: | ATGTGGGGAGCTACTCATCCC | (gene nt.2523-2503) |
| SEQ ID No.60: | GGGATGAGTAGCTCCCCACAT**gcc**CTAAGAAACAG | (V823A) |
| SEQ ID No.61: | GGGATGAGTAGCTCCCCACAT**tgc**CTAAGAAACAG | (V823C) |
| SEQ ID No.62: | GGGATGAGTAGCTCCCCACAT**gac**CTAAGAAACAG | (V823D) |
| SEQ ID No.63: | GGGATGAGTAGCTCCCCACAT**gag**CTAAGAAACAG | (V823E) |
| SEQ ID No.64: | GGGATGAGTAGCTCCCCACAT**ggg**CTAAGAAACAG | (V823G) |
| SEQ ID No.65: | GGGATGAGTAGCTCCCCACAT**cac**CTAAGAAACAG | (V823H) |
| SEQ ID No.66: | GGGATGAGTAGCTCCCCACAT**aag**CTAAGAAACAG | (V823K) |
| SEQ ID No.67: | GGGATGAGTAGCTCCCCACAT**aac**CTAAGAAACAG | (V823N) |
| SEQ ID No.68: | GGGATGAGTAGCTCCCCACAT**ccc**CTAAGAAACAG | (V823P) |
| SEQ ID No.69: | GGGATGAGTAGCTCCCCACAT**cag**GTAAGAAACAG | (V823Q) |
| SEQ ID No.70: | GGGATGAGTAGCTCCCCACAT**cgc**CTAAGAAACAG | (V823R) |
| SEQ ID No.71: | GGGATGAGTAGCTCCCCACAT**agc**CTAAGAAACAG | (V823S) |
| SEQ ID No.72: | GGGATGAGTAGCTCCCCACAT**acc**CTAAGAAACAG | (V823T) |

The mutagenesis was done using the same general procedure described for mutagenesis of peptide 10 (above), except that the 5' fragment was 310bp in length and the 3'fragment 606bp. The joined fragment was 894bp in length and was digested with AvrII and PflMI and cloned into similarly digested pCIF8.
One clone with the correct sequence was selected for each mutant and their activities and expression levels analysed as described above. Transfections were done in duplicate and supernatants were pooled prior to assay.
Comparison of expression levels and activity for the above mutants revealed that a variety of changes could be made without substantially affecting expression levels and activity. These data are depicted in FIGURE 7. Alteration of V823 to A, D, E, G, H, N, P, S or T yielded mutants with expression and activity at least equivalent to that of wt factor VIII. Therefore these mutations are useful for the removal ofT cell epitopes associated with peptide 7.

### EXAMPLE 3

FVIII derived peptides were synthesised containing mutations and tested for their continued ability to promote T-cell proliferation using an ex vivo assay. The peptides were 15mer sequences and were designed to test the substitutions I1011A or I1011T in combination with M1013K. The peptides were tested using 4 PBMC donor samples shown previously to be responsive to the wild-type peptide sequence. In all instances the mutant peptides tested were unable to stimulate proliferation with an SI > 2.0. Results of this assay including allotype details of the donors and peptide sequences are shown in FIGURE 8.
PBMC were stimulated with protein and peptide antigens in a 96 well flat bottom plate at a density of 2x10⁶ PBMC per well. PBMC were incubated for 7 days at 37°C before pulsing with ³H-Thymidine. Peptides were generated with the specified substitutions and each peptide was screened individually against PBMC's isolated from 4 healthy donors shown previously to be responsive to the FVIII P8 peptide sequence. A control peptide from influenza haemagglutinin and a potent non-recall antigen keyhole limpet haemocyanin (KLH) were used in each donor assay.
Peptides were dissolved in DMSO to a final concentration of 10mM, these stock solutions were then diluted 1/500 in AIM V media (final concentration 20µM). Peptides were added to a flat bottom 96 well plate to give a final concentration of 2 and 20µM in a 100µl. The viability of thawed PBMC's was assessed by trypan blue dye exclusion, cells were then resuspended at a density of 2x10⁶ cells/ml, and 100µl (2x10⁵ PBMC/well) was transferred to each well containing peptides. Triplicate well cultures were assayed at each peptide concentration. Plates were incubated for 7 days in a humidified atmosphere of 5% CO₂ at 37°C. Cells were pulsed for 18-21 hours with 1µCi ³H-Thy/well before harvesting onto filter mats. CPM values were determined using a Wallac microplate beta top plate counter (Perkin Elmer). Results were expressed as stimulation indices, calculated by dividing the CPM value to the test peptide by the CPM value in the untreated wells. A stimulation index of greater than 2 is taken as positive proliferation.

## Claims

1. A modified human Factor VIII molecule being non-immunogenic or less immunogenic than non-modified human Factor VIII and having the same biological specificity and activity when used in vivo, comprising an amino acid residue substitution compared with the non-modified parental molecule, wherein said amino acid residue substitution causes a reduction or an elimination of one or more T-cell epitopes which act in the parental non-modified molecule as MHC class II binding ligands and stimulate T-cells, wherein said amino acid residue substitution is selected from the group consisting of V823A, V823D, V823G, V823H, V823N, V823S, V823T.

2. A modified Factor VIII molecule according to claim 1, which exhibits, when tested in a biological assay of induced cellular proliferation of human T-cells a stimulation index (SI smaller than 2 and smaller than the parental Factor VIII molecule tested in parallel using cells from the same donor, wherein said index is taken as the value of cellular proliferation scored following stimulation by the Factor VIII molecule divided by the proliferation scored in control cells not contacted with the Factor VIII molecule, and wherein cellular proliferation is measured by any suitable means.

3. A DNA sequence coding for a Factor VIII molecule as defined in claim 1 or 2.

4. A pharmaceutical composition comprising a modified Factor VIII molecule of claim 1 or 2, optionally together with a pharmaceutically acceptable carrier, diluent or excipient.

## Patentansprüche

1. Modifiziertes humanes Faktor-VIII-Molekül, das nicht immunogen oder weniger immunogen als der nicht modifizierte, humane Faktor VIII ist und die gleiche biologische Spezifizität und Aktivität bei Verwendung in vivo aufweist, enthaltend eine Aminosäurerestsubstitution verglichen mit dem nicht modifizierten parentalen Molekül, wobei diese Aminosäurerestsubstitution eine Verminderung oder eine Eliminierung von einem oder mehreren T-Zell-Epitopen verursacht, die in dem parentalen, nicht modifizierten Molekül als MHC-Klasse-II-bindende Liganden wirken und T-Zellen stimulieren, wobei diese Aminosäurerestsubstitution ausgewählt wird aus der Gruppe bestehend aus: V823A, V823D, V823G, V823H, V823N, V823S, V823T.

2. Modifiziertes Faktor-VIII-Molekül nach Anspruch 1, das bei der Untersuchung in einem biologischen Assay für induzierte zelluläre Proliferation von humanen T-Zellen einen Stimulationsindex (SI) von kleiner als 2 und kleiner als das parentale Faktor-VIII-Molekül aufweist, das gleichzeitig unter Verwendung von Zellen vom gleichen Spender untersucht wurde, wobei dieser Index als der Wert der zellulären Proliferation, der nach Stimulierung durch das Faktor-VIII-Molekül erreicht wurde, geteilt durch die Proliferation, die in nicht mit dem Faktor-VIII-Molekül in Kontakt stehenden Kontrollzellen erreicht wurde, genommen wird und wobei die zelluläre Proliferation durch jegliches geeignete Mittel gemessen wird.

3. DNA-Sequenz, kodierend für ein Faktor-VIII-Molekül nach Anspruch 1 oder 2.

4. Pharmazeutische Zusammensetzung, enthaltend ein modifiziertes Faktor-VIII-Molekül nach Anspruch 1 oder 2, gegebenenfalls zusammen mit einem pharmazeutisch unbedenklichen Träger, Verdünnungsmittel oder Hilfsstoff.

## Revendications

1. Molécule de facteur VIII humain modifiée qui est non immunogène ou moins immunogène que celle de facteur VIII humain non modifiée et qui présente la même spécificité biologique et la même activité biologique que lors d'un usage in vivo, comprenant une substitution de résidu d'acide aminé par comparaison avec la molécule parentale non modifiée, dans laquelle ladite substitution de résidu d'acide aminé provoque une réduction ou une élimination d'un ou de plusieurs épitopes de lymphocyte T qui agissent dans la molécule parentale non modifiée en tant que ligands de liaison MHC classe II et qui stimulent les lymphocytes T, dans laquelle ladite substitution de résidu d'acide aminé est choisie parmi le groupe comprenant : V823A, V823D, V823G, V823H, V823N, V823S, V823T.

2. Molécule de facteur VIII modifiée selon la revendication 1, qui présente, lorsqu'elle est testée dans un essai biologique de prolifération cellulaire induite de lymphocytes T humains, un indice de stimulation (SI) inférieur à 2 et inférieur à celui de la molécule de facteur VIII parentale testée en parallèle en utilisant des cellules provenant du même donneur, dans laquelle ledit indice est pris en tant que valeur de la prolifération cellulaire décomptée suite à une stimulation par la molécule de facteur VIII divisée par la prolifération décomptée pour des cellules de contrôle non mises en contact avec la molécule de facteur VIII, et dans laquelle la prolifération cellulaire est mesurée par un quelconque moyen approprié.

3. Séquence d'ADN codant pour une molécule de facteur VIII selon la revendication 1 ou 2.

4. Composition pharmaceutique comprenant une molécule de facteur VIII modifiée selon la revendication 1 ou 2, en option associée à un vecteur, diluent ou excipient acceptable pharmaceutiquement.
